# EUROPÄISCHE PATENTSCHRIFT

(11) **EP 1 734 048 B1**
(45) Veröffentlichungstag und Bekanntmachung des Hinweises auf die Patenterteilung: **17.12.2008**
(21) Anmeldenummer: 06015805.2
(22) Anmeldetag: 27.03.2003
(51) Int. Cl.: C07K 7/06, C07K 16/00, C12N 15/00, A61K 38/00

(54) **An MHC-Moleküle bindende Tumor-assoziierte Peptide**
Tumor-associated peptides that bind to MHC-molecules
Peptides associés à une tumeur et se liant à des molecules MHC

(30) Priorität: 29.05.2002 DE 10225144
(43) Veröffentlichungstag der Anmeldung: 20.12.2006
(62) Teilanmeldung aus: 03720376.7
(73) Patentinhaber: Immatics Biotechnologies GmbH, 72076 Tübingen (DE)
(72) Erfinder: Weinschenk, Toni, 73730 Esslingen (DE); Rammensee, Hans-Georg, 72070 Tübingen-Unterjesingen (DE); Stevanovic, Stefan, 72074 Tübingen (DE)
(74) Vertreter: Krauss, Jan

(56) Entgegenhaltungen:
- WEINSCHENK T ET AL: "Integrated functional genomics approach for the design of patient-individual antitumor vaccines" CANCER RESEARCH, AMERICAN ASSOCIATION FOR CANCER RESEARCH, BALTIMORE, MD, US, Bd. 62, Nr. 20, 15. Oktober 2002 (2002-10-15), Seiten 5818-5827, XP002266492 ISSN: 0008-5472
- SCHIRLE MARKUS ET AL: "Identification of tumor-associated MHC class I ligands by a novel T cell-independent approach" EUROPEAN JOURNAL OF IMMUNOLOGY, WEINHEIM, DE, Bd. 30, Nr. 8, August 2000 (2000-08), Seiten 2216-2225, XP002246625 ISSN: 0014-2980

## Beschreibung

Die vorliegende Erfindung betrifft Tumor-assoziierte Peptide, die die Fähigkeit aufweisen, an ein Molekül des menschlichen Haupt-Histokompatibilitäts-Komplexes (MHC), Klasse I, zu binden.

Derartige Peptide werden bspw. in der Immuntherapie von Tumorerkrankungen eingesetzt.

Bei der Eliminierung von Tumorzellen durch das Immunsystem spielt die Erkennung von Tumor-assoziierten Antigenen (TAA) durch Komponenten des Immunsystems eine herausragende Rolle. Diesem Mechanismus liegt die Voraussetzung zugrunde, dass zwischen Tumorzellen und normalen Zellen qualitative oder quantitative Unterschiede bestehen. Um eine anti-Tumor-Antwort herbeizuführen, müssen die Tumorzellen Antigene exprimieren, gegen welche eine immunologische Antwort erfolgt, die für die Eliminierung des Tumors ausreicht.

Beteiligt bei der Abstoßung von Tumoren sind insbesondere CD8-exprimierende zytotoxische T-Lymphozyten (im folgenden CTL). Zur Auslösung einer derartigen Immunreaktion durch zytotoxische T-Zellen müssen den T-Zellen dazu fremde Proteine/Peptide präsentiert werden. T-Zellen erkennen Antigene als Peptidfragmente nur dann, wenn diese an Zelloberflächen von MHC-Molekülen präsentiert werden. Diese MHC-Moleküle ("major histocompatibility complex") sind Peptidrezeptoren, die normalerweise Peptide innerhalb der Zelle binden, um sie zu der Zelloberfläche zu transportieren. Dieser Komplex aus Peptid und MHC-Molekül kann durch die T-Zellen erkannt werden. Die MHC-Moleküle des Menschen werden auch als humane Leukozyten-Antigene (HLA) bezeichnet.

Es gibt zwei Klassen von MHC-Molekülen: MHC-Klasse-I-Moleküle, die auf den meisten Zellen mit Kern vorkommen, präsentieren Peptide, die durch proteolytischen Abbau endogener Proteine entstehen. MHC-Klasse-II-Moleküle kommen nur auf professionellen Antigen-präsentierenden Zellen (APC) vor und präsentieren Peptide exogener Proteine, die im Verlauf der Endozytose von APC aufgenommen und verarbeitet werden. Komplexe aus Peptid und MHC-Klasse-I werden von CD8-positiven zytotoxischen T-Lymphozyten erkannt, Komplexe aus Peptid und MHC-Klasse-II werden von CD4-Helfer-T-Zellen erkannt.

Damit ein Peptid eine zelluläre Immunantwort auslösen kann, muss es an ein MHC-Molekül binden. Dieser Vorgang ist vom Allel des MHC-Moleküls und von der Aminosäuresequenz des Peptids abhängig. MHC-Klasse-I-bindende Peptide sind in der Regel 8-10 Reste lang und enthalten in ihrer Sequenz zwei konservierte Reste ("Anker"), die mit der entsprechenden Bindungsfurche des MHC-Moleküls interagieren.

Damit das Immunsystem eine effektive CTL-Antwort gegen Tumor-abgeleitete Peptide starten kann, müssen diese Peptide nicht nur in der Lage sein, an die bestimmten MHC-Klasse-I-Moleküle zu binden, die von den Tumorzellen exprimiert werden, sondern sie müssen auch von T-Zellen mit spezifischen T-Zell-Rezeptoren (TCR, "T-cell receptor") erkannt werden.

Das Hauptziel zur Entwicklung einer Tumorvakzine ist die Identifizierung und Charakterisierung von Tumor-assoziierten Antigenen, die durch CD8⁺ CTL erkannt werden.

Die Antigene, die von den Tumor-spezifischen zytotoxischen T-Lymphozyten erkannt werden, bzw. deren Epitope, können Moleküle aus sämtlichen Proteinklassen sein, wie z.B. Enzyme, Rezeptoren, Transkriptionsfaktoren, etc. Eine andere wichtige Klasse Tumorassoziierter Antigene sind Gewebe-spezifische Strukturen, wie bspw. CT ("cancer testis")-Antigene, die in verschiedenen Tumorarten und in gesundem Hodengewebe exprimiert werden.

Damit die Proteine durch die zytotoxischen T-Lymphozyten als Tumor-spezifisches Antigen erkannt werden, und um somit in einer Therapie eingesetzt werden zu können, müssen bestimmte Voraussetzungen vorliegen: Das Antigen soll hauptsächlich von Tumorzellen exprimiert werden und von Normalgeweben nicht oder nur in geringeren Mengen als in den Tumoren. Weiterhin ist wünschenswert, wenn das betreffende Antigen nicht nur in einer Tumorart, sondern auch in weiteren in hoher Konzentration vorliegt. Unbedingt essentiell ist auch das Vorliegen von Epitopen in der Aminosäuresequenz des Antigens, da solche von einem Tumor-assoziierten Antigen abgeleiteten Peptide ("immunogene Peptide") zu einer T-Zell-Antwort führen sollen, sei es *in vitro* oder *in vivo.*

TAAs stellen somit ein Ausgangspunkt für die Entwicklung einer Tumorvakzine dar. Die Methoden zur Identifizierung und Charakterisierung der TAA beruhen zum einen auf dem Einsatz von in Patienten bereits induzierten CTL oder basieren auf der Erstellung differentieller Transkriptionsprofile zwischen Tumoren und Normalgeweben.

Das Auffinden von Genen, die in Tumorgeweben überexprimiert sind, oder die in derartigen Geweben selektiv exprimiert werden, liefert jedoch keine präzise Information für einen Einsatz der von diesen Genen transkribierten Antigene in der Immuntherapie. Dies hat die Ursache darin, dass jeweils nur einzelne Epitope dieser Antigene für einen derartigen Einsatz geeignet sind, da nur die Epitope der Antigene - nicht das gesamte Antigen - durch MHC-Präsentierung eine T-Zell-Antwort hervorrufen. Daher ist es wichtig, diejenigen Peptide von überexprimierten oder selektiv exprimierten Proteinen zu selektieren, die mit MHC-Molekülen präsentiert werden, wodurch Angriffspunkte für die spezifische Tumorerkennung durch zytotoxische T-Lymphozyten gewonnen werden könnten.

Vor diesem Hintergrund ist es Aufgabe der vorliegenden Erfindung, mindestens eine neue Aminosäuresequenz für ein derartiges Peptid bereitzustellen, welches die Fähigkeit aufweist, an ein Molekül des menschlichen Haupt-Histokompatibilitäts-Komplexes (MHC)-Klasse-I zu binden.

Diese Aufgabe wird erfindungsgemäß gelöst durch die Bereitstellung eines Tumor-assoziierten Peptids mit einer Aminosäuresequenz, die ausgewählt ist aus der Gruppe bestehend aus SEQ ID-Nr. 1 bis SEQ ID-Nr. 79 aus dem beiliegenden Sequenzprotokoll, wobei das Peptid die Fähigkeit aufweist, an ein Molekül des menschlichen Haupt-Histokompatibilitäts-Komplexes (MHC) Klasse-I zu binden.

Die der Erfindung zugrunde liegende Aufgabe wird auf diese Weise vollkommen gelöst.

Dabei versteht sich, dass die vom Tumor identifizierten Peptide zur Gewinnung größerer Mengen und zum Einsatz bei den unten aufgeführten Zwecken synthetisiert oder in Zellen zur Expression gebracht werden.

Die Erfinder konnten die obengenannten Peptide als spezifische Liganden von MHC-Klasse-I-Molekülen aus Tumorgewebe isolieren und identifizieren. Dabei werden hierin mit dem Begriff "Tumor-assoziiert" Peptide bezeichnet, die aus Tumormaterial isoliert und identifiziert wurden. Diese Peptide, die auf echten (primären) Tumoren präsentiert werden, unterliegen also der Antigenprozessierung in einer Tumor-Zelle.

Die spezifischen Liganden können in der Krebstherapie eingesetzt werden, z.B. um eine Immunantwort gegen Tumorzellen zu induzieren, die die entsprechenden Antigene exprimieren, von denen die Peptide abstammen.

Eine solche Immunantwort in Form einer Induktion von CTL kann zum einen *in vivo* erreicht werden. Dazu wird einem Patienten, der an einer mit dem TAA assoziierten Tumorerkrankung leidet, das Peptid bspw. in Form einer pharmazeutischen Zusammensetzung verabreicht.

Andererseits kann eine CTL-Antwort auf einen Tumor, der die Antigene, von denen die Peptide abstammen, exprimiert, auch *ex vivo* ausgelöst werden. Dazu inkubiert man die CTL-Vorläuferzellen zusammen mit Antigen-präsentierenden Zellen und den Peptiden. Anschließend kultiviert man die dadurch stimulierten CTL und verabreicht diese aktivierten CTL dem Patienten.

Weiterhin besteht die Möglichkeit, APC *ex vivo* mit den Peptiden zu beladen und diese beladenen APC dem Patienten zu verabreichen, der im Tumorgewebe das Antigen exprimiert, von dem das Peptid abstammt. Die APC können dann wiederum *in vivo* den CTL das Peptid präsentieren und sie aktivieren.

Die erfindungsgemäßen Peptide können aber auch als diagnostische Reagenzien eingesetzt werden.

So kann mit den Peptiden herausgefunden werden, ob in einer CTL-Population spezifisch gegen ein Peptid gerichtete CTL vorliegen oder durch eine Therapie induziert wurden.

Außerdem kann mit den Peptiden die Zunahme von Vorläufer T-Zellen getestet werden, die eine Reaktivität gegen das definierte Peptid aufweisen.

Ferner kann das Peptid als Marker dazu verwendet werden, um den Krankheitsverlauf eines Tumors zu verfolgen, der das Antigen exprimiert, von dem das Peptid abstammt.

In der beigefügten Tabelle 1 sind die identifizierten Peptide aufgeführt. Dabei sind sie nach den jeweiligen HLA-Typen, an die sie binden, geordnet. In der Tabelle sind außerdem die Proteine angegeben, von denen das Peptid abstammt und die jeweilige Position des Peptids in dem betreffenden Protein. Dabei sind die englischen Bezeichnungen der Proteine beibehalten worden, um missverständliche Übersetzungen zu vermeiden. Ferner sind jeweils die Acc-Nummern angegeben, die in der Genbank des "National Center for Biotechnology Information" des National Institute of Health geführt werden (siehe http:\\www.ncbi.nlm.nih.gov).

Die Erfinder konnten die Peptide (oder Liganden) aus Nierenzelltumoren zweier Patienten, RCC01 und RCC13, isolieren. Dabei wurden 68 Liganden aus Tumorgewebe des Patienten RCC01 isoliert, 13 aus Tumorgewebe des Patienten RCC13. Zwei der in beiden Patienten identifizierten Liganden waren identisch. Dies waren die Peptide mit der SEQ ID-Nr. 1 und 3 (YVDPVITSI von met-Protoonkogen (C-Met) und ALLNIKVKL von Keratin 18).

Aus den Tumoren der Patienten konnten 79 Liganden identifiziert werden, von denen 30 an den HLA-Subtypen HLA-A*02, 13 an HLA-A*68, 34 an HLA-B*18 oder HLA-B*44 und 2 an HLA*24 gebunden waren.

HLA-A*02-Liganden wiesen alle das allel-spezifische Peptidmotiv auf: (Leucin/Valin, Isoleucin, Alanin oder Methionin an Position 2, Leucin/Valin, Isoleucin oder Alanin am C-Terminus.

Einige der Liganden stammten von stark exprimierten sogenannten "Housekeeping" Genen ab, die in den meisten Geweben gleichmäßig exprimiert werden, viele zeichneten sich aber durch Tumor-Assoziierung aus.

Bei dem Peptid mit der Sequenz ID-Nr. 1 YVDPVITSI handelt es sich z.B. um einen Liganden, der insbesondere mit Tumoren in Verbindung gebracht wird und der vom met-Protoonkogen (c-Met) (Position 654-662) abstammt. Die Peptide mit der Sequenz ID-Nr. 2, 22 und 23 stammen vom Adipophilin (auch als "Adipose differentiation related" Protein bezeichnet) ab und weisen die Positionen 129-137, 62-71 und 349-358 in diesem Protein auf, wobei die letzteren beiden zu den durch HLA-A*68 präsentierten Peptiden zählten. Bei dem Liganden mit der Sequenz ID-Nr. 3 handelt es sich um einen Liganden, der von Keratin 18 herrührt und dort bei Position 365-373 lokalisiert ist.

Der größte Teil der Liganden wies an Position 2 die Aminosäure Glutaminsäure (E) auf, eine Anker-Aminosäure des HLA-B*44-Subtyps. So konnten Peptide identifiziert werden, die von Proteinen abstammen, die sich bereits in früheren Versuchen als immunogen erwiesen haben, wie bspw. das Peptid mit der Sequenz ID-Nr. 5, welches von dem Protein Annexin II abstammt (Position in Annexin II: 55-63). Dieses Protein erwies sich in bezug auf MHC Klasse II-Moleküle in Melanom-Patienten als immunogen (siehe Heinzel et al., The self peptide annexin II (208 - 223) presented by dendritic cells sentisizes autologous CD4+ T lymphocytes to recognize melanoma cells, 2001, Cancer Immunol. Immunother. 49:671-678).

Außerdem konnten einige Peptide identifiziert werden, die von Proteinen abstammen, die insbesondere in Tumorgewebe überexprimiert werden. So konnten Fragmente von Vimentin (EEIAFLKKL, Position 229-237) und Caldesmon (DEAAFLERL, Position 92-100) identifiziert werden. Young et al., Expression profiling of renal epithelial neoplasms: a method for tumor classification and discovery of diagnostic molecular markers, 2001, Am. J. Pathol., 158:1639-1651) zeigten, dass diese Proteine in Gewebe von Nierenzelltumoren überexprimiert waren.

Die Erfinder konnten außerdem u.a. Liganden identifizieren, die von ets-1 (NEFSLKGVDF, Position 86-95), Alpha-Catenin (NEQDLGIQY, Position 169-177) und Galectin 2 (SEVKFTVTF, Position 80-88) abstammen.

Weiterhin isolierten die Erfinder das Fragment YYMIGEQKF (Sequenz ID-Nr. 79), welches von dem Enzym Nicotinamid-N-Methyltransferase abstammt (Position 203-211). Takahashi et al., Gene expression profiling of clear cell renal cell carcinoma: gene identification and prognostic classification, 2001, Proc. Natl. Acad. Sci. USA, 98:9754-9749, zeigten, dass dieses Enzym in Nierenzelltumorgewebe überexprimiert war.

Die Erfinder konnten überraschenderweise für eines der identifizierten Peptide spezifische zytotoxische T-Lymphozyten in Spenderblut nachweisen. Dadurch ist das Potential gegeben, um eine spezifische CTL-Antwort gegen die Tumoren zu triggern.

Ferner konnten die Erfinder in eigenen Versuchen zeigen, dass es unter Verwendung zweier beispielhaft ausgewählter Peptide möglich war, *in vitro* zytotoxische T-Lymphözyten (CTL) zu generieren, die spezifisch für das Peptid mit der SEQ ID-Nr. 1 (c-Met-Protoonkogen-Fragment oder c-Met-Peptid) oder spezifisch für das Peptid mit der SEQ ID-Nr. 2 (Adipophilin-Fragment oder Adipophilin-Peptid) waren. Mit diesen CTL konnten gezielt Tumorzellen abgetötet werden, welche die entsprechenden Proteine exprimierten und welche darüber hinaus von verschiedenen Tumor-Zelllinien unterschiedlicher Patienten stammten. Die Erfinder konnten weiterhin zeigen, dass die genannten CTL auch bspw. dendritische Zellen lysierten, die zuvor mit den jeweiligen Peptiden "gepulst" (beladen) wurden. Durch diese Versuche konnten die Erfinder zeigen, dass mit den erfindungsgemäßen Peptiden als Epitope humane T-Zellen *in vitro* aktiviert werden konnten. Die Erfinder konnten demnach nicht nur zeigen, dass CTL, die aus peripheren Blut-mononukleären-Zellen (PBMNC) eines Patienten gewonnen wurden und die für ein bestimmtes Peptid spezifisch waren, Zellen der gleichen Tumorart eines anderen Patienten abtöten konnten. Die Erfinder zeigten außerdem, dass mit diesen CTL auch Zellen anderer Tumorarten lysiert werden konnten.

In einer bevorzugten Ausführungsform können auch Peptide zur Stimulierung einer Immunantwort eingesetzt werden, die die Sequenz ID-Nr. 1 bis 79 aufweisen und bei denen zumindest eine Aminosäure durch eine andere Aminosäure mit ähnlichen chemischen Eigenschaften ersetzt ist.

Dies sind mit Bezug auf die jeweiligen MHC-Subtypen bspw. die Ankeraminosäuren, die durch Aminosäuren mit ähnlichen chemischen Eigenschaften ersetzt werden können. So kann bspw. bei Peptiden, die mit dem MHC-Subtyp HLA-A*02 assoziiert sind, an Position 2 Leucin mit Isoleucin, Valin oder mit Methionin und umgekehrt ausgetauscht werden, und am C-Terminus Leucin mit Valin, Isoleucin und Alanin, die allesamt unpolare Seitenketten aufweisen.

Weiterhin ist es möglich, Peptide mit der Sequenz ID-Nr. 1 bis 79 einzusetzen, die N- oder/und C-terminal zumindest eine weitere Aminosäure aufweisen oder bei denen zumindest eine Aminosäure deletiert ist.

Weiterhin können Peptide mit der Sequenz ID-Nr. 1 bis 79 eingesetzt werden, bei denen zumindest eine Aminosäure chemisch modifiziert ist.

Die variierende(n) Aminosäure(n) ist(sind) dabei so gewählt, dass durch die Variation die Immunogenität des Peptids nicht beeinträchtigt ist, d.h. eine ähnliche Bindungsaffinität an das MHC-Molekül und die Fähigkeit zur T-Zell-Stimulierung aufweist.

Erfindungsgemäß kann das Peptid zur Behandlung von Tumorerkrankungen und/oder adenomatöser Erkrankungen verwendet werden.

Die zu behandelnden Tumorerkrankungen umfassen dabei bspw. Nieren-, Brust-, Pankreas-, , Magen-, Hoden- und/oder Hautkrebs. Die Aufzählung der Tumorerkrankungen ist dabei lediglich beispielhaft und soll den Verwendungsbereich nicht eingrenzen.

Dass die erfindungsgemäßen Peptide für eine solche Verwendung geeignet sind, konnten die Erfinder in eigenen Versuchen zeigen. Darin wurde nachgewiesen, dass eigens generierte CTL, die spezifisch für bestimmte Peptide waren, effektiv und selektiv Tumorzellen abtöten konnten.

Für einen Einsatz von Tumor-assoziierten Antigenen in einer Tumorvakzine sind grundsätzlich mehrere Applikationsformen möglich. So beschrieben Tighe et al., 1998, Gene vaccination: plasmid DNA is more than just a blueprint, Immunol. Today 19(2):89-97, dass das Antigen entweder als rekombinantes Protein mit geeigneten Adjuvantien bzw. Trägersystemen oder als die für das Antigen kodierende cDNA in Plasmidvektoren verabreicht werden kann. In diesen Fällen muss das Antigen im Körper des Patienten von Antigen-präsentierenden Zellen (APC) verarbeitet und präsentiert werden, damit eine Immunantwort ausgelöst wird.

Melief et al., 1996, Peptide-based cancer vaccines, Curr. Opin. Immunol. 8:651-657, zeigen eine weitere Möglichkeit, nämlich die Verwendung von synthetischen Peptiden als Vakzine.

Das Peptid kann dabei in einer bevorzugten Ausführungsform mit Zugabe von Adjuvantien verwendet werden, oder aber auch in Alleinstellung.

Als Adjuvans kann bspw. der Granulocyte-macrophage-colony-stimulating-factor (GM-CSF) eingesetzt werden.

Weitere Beispiele für solche Adjuvantien sind Aluminiumhydroxid, Emulsionen von Mineralölen, wie bspw. das Freund'sche Adjuvans, Saponine oder Siliciumverbindungen.

Die Verwendung mit Adjuvantien bietet den Vorteil, dass die durch das Peptid ausgelöste Immunantwort verstärkt werden kann und/oder dass das Peptid stabilisiert wird.

In einer anderen bevorzugten Ausführungsform wird das Peptid gebunden auf einer Antigen-präsentierenden Zelle eingesetzt.

Diese Maßnahme hat den Vorteil, dass die Peptide dem Immunsystem, insbesondere den zytotoxischen T-Lymphozyten (CTL), präsentiert werden können. Dadurch können die CTL die Tumorzellen erkennen und spezifisch abtöten. Als Antigen-präsentierende Zellen sind bspw. dendritische Zellen, Monozyten oder B-Lymphozyten für einen solchen Einsatz geeignet.

Dabei werden die Zellen bspw. *ex vivo* mit den Peptiden beladen. Andererseits besteht auch die Möglichkeit, die Zellen mit der für die Peptide kodierenden DNA oder der entsprechenden RNA zu transfizieren, um dann die Peptide auf den Zellen zur Expression zu bringen.

Die Erfinder, konnten in eigenen Versuchen zeigen, dass es möglich ist, dendritische Zellen (DC) mit spezifischen Peptiden zu beladen, und dass diese beladenen dendritischen Zellen Peptid-spezifische CTL aktivieren. Dies bedeutet, dass das Immunsystem stimuliert werden kann, CTL gegen die Tumore zu entwickeln, welche die entsprechenden Peptide exprimieren.

Die das Peptid tragenden Antigen-präsentierenden Zellen können dabei entweder direkt verwendet werden oder aber vor einem Einsatz bspw. mit dem Hitzeschock-Protein gp96 aktiviert werden. Dieses Hitzeschock-Protein induziert die Expression von MHC-Klasse I-Molekülen und von costimulierenden Molekülen wie B7 und stimuliert außerdem die Produktion von Zytokinen. Dadurch wird insgesamt die Auslösung von Immunantworten gefördert.

In einer anderen bevorzugten Ausführungsform werden die Peptide zur Markierung von Leukozyten, insbesondere von T-Lymphozyten verwendet.

Diese Verwendung ist von Vorteil, wenn mit den Peptiden herausgefunden werden soll, ob in einer CTL-Population spezifisch gegen ein Peptid gerichtete CTL vorliegen.

Ferner kann das Peptid als Marker zur Beurteilung eines Therapieverlaufes bei einer Tumorerkrankung verwendet werden.

Auch bei anderen Immunisierungen oder Therapien kann das Peptid für das Monitoring der Therapie eingesetzt werden. Somit ist das Peptid nicht nur therapeutisch, sondern auch diagnostisch einsetzbar.

In einer weiteren Ausführungsform werden die Peptide zur Herstellung eines Antikörpers eingesetzt.

Polyklonale Antikörper können in herkömmlicher Weise durch Immunisierung von Tieren mittels Injektion der Peptide und anschließender Reinigung des Immunglobulins gewonnen werden.

Monoklonale Antikörper können nach Standardprotokollen hergestellt werden, wie bspw. in Methods Enzymol. (1986), 121, Hybridoma technology and monoclonal antibodies, beschrieben.

Die Erfindung betrifft außerdem in einem weiteren Aspekt eine pharmazeutische Zusammensetzung, die eines oder mehrere der Peptide enthält.

Diese Zusammensetzung dient bspw. der parenteralen Verabreichung, bspw. subkutan, intradermal oder intramuskulär, oder der oralen Verabreichung. Dabei sind die Peptide in einem pharmazeutisch annehmbaren, vorzugsweise wässrigen, Träger gelöst oder suspendiert. Darüber hinaus kann die Zusammensetzung Hilfsstoffe, wie bspw. Puffer, Bindemittel, Verdünnungsmittel, etc. enthalten.

Die Peptide können auch zusammen mit immunstimulierenden Substanzen, z.B. Zytokinen, verabreicht werden. Eine umfassende Darstellung von Hilfsstoffen, wie sie bei einer derartigen Zusammensetzung verwendet werden können, ist bspw. in A. Kibbe, Handbook of Pharmaceutical Excipients, 3. Ed., 2000, American Pharmaceutical Association and pharmaceutical press, dargestellt.

Das Mittel kann dabei zur Prävention, Prophylaxe und/oder Therapie von Tumorerkrankungen und/oder adenomatöser Erkrankungen eingesetzt werden.

Das pharmazeutische Mittel, das zumindest eines der Peptide mit der Sequenz ID-Nr. 1 bis 79 enthält, wird einem Patienten verabreicht, der an einer Tumorerkrankung leidet, mit der das betreffende Peptid bzw. Antigen assoziiert ist. Dadurch kann eine Tumor-spezifische Immunantwort auf Basis Tumor-spezifischer CTL ausgelöst werden.

Die in der pharmazeutischen Zusammensetzung vorliegende Menge des Peptids oder der Peptide liegt dabei in einer therapeutisch effektiven Menge vor. Dabei können die in der Zusammensetzung enthaltenen Peptide auch an mindestens zwei verschiedene HLA-Typen binden.

Die vorliegende Erfindung betrifft in einem weiteren Aspekt Nukleinsäuremoleküle, die für die Peptide mit der Sequenz ID-Nr. 1 bis 79 kodieren.

Die Nukleinsäuremoleküle können dabei DNA- oder RNA-Moleküle sein und ebenfalls für die Immuntherapie von Krebserkrankungen eingesetzt werden. Dabei induziert das von dem Nukleinsäuremolekül exprimierte Peptid eine Immunantwort gegen Tumorzellen, die das Peptid exprimieren.

Erfindungsgemäß können die Nukleinsäuremoleküle auch in einem Vektor vorliegen.

Darüber hinaus betrifft die Erfindung Zellen, die mit Hilfe des Nukleinsäuremoleküls, welches für die Peptide kodiert, genetisch derart verändert wurde, dass sie ein Peptid mit der Sequenz ID-Nr. 1 bis 79 produziert.

Die Zellen werden hierfür mit der für die Peptide kodierenden DNA oder der entsprechenden RNA transfiziert, wodurch die Peptide auf den Zellen zur Expression gebracht werden. Für einen solchen Einsatz sind als Antigen-präsentierende Zellen bspw. dendritische Zellen, Monozyten oder B-Lymphozyten geeignet.

Es versteht sich, dass die vorstehend genannten und die nach-stehend noch zu erläuternden Merkmale nicht nur in der jeweils angegebenen Kombination, sondern auch in Alleinstellung verwendbar sind, ohne den Rahmen der vorliegenden Erfindung zu verlassen.

Ausführungsbeispiele der Erfindung werden in den nachfolgenden Beispielen und den beigefügten Figuren dargestellt und erläutert. Es zeigen:
- Fig. 1: die Detektion von Keratin 18-spezifischen CD8⁺-T-Lymphozyten;
- Fig. 2a-d: die Induktion von c-Met- (SEQ ID-Nr. 1), Fig. 2a+b oder Adipophilin- (SEQ ID-Nr. 2), Fig. 2c+d, Peptid-spezifischen CTL-Antworten *in vitro*;
- Fig. 3a-f: die Antigen-spezifische Lyse von c-Met- oder Adipophilin-exprimierenden Tumor-Zelllinien durch CTL, induziert durch c-Met- (SEQ ID-Nr. 1), Fig. 3a-d, oder Adipophilin- (SEQ ID Nr. 2), Fig. 3e-f, Peptid-induzierten CTL;
- Fig. 4a-c: Lysis-Inhibitionsassays mit ⁵¹Cr-markierten Tumorzellen und unmarkierten, Peptid-gepulsten T2-Zellen durch c-Met- (SEQ ID-Nr. 1), Fig. 4a+b, oder Adipophilin-(SEQ ID-Nr. 2), Fig. 4c, Peptid-induzierten CTL;
- Fig. 5a+b: die Lyse von autologen, mit Tumor-RNA transfizierten DC durch c-Met-(SEQ ID-Nr. 1), Fig. 5a, oder Adipophilin-(SEQ ID-Nr. 2), Fig. 5b, Peptid induzierte CTL;
- Fig. 6: *in*-*vitro*-induzierte Adipophilin-spezifische autologe CTL erkennen autologe Tumorzellen eines Patienten mit chronisch lymphatischer Leukämie, aber nicht autologe dendritische oder B-Zellen.

### Beispiel 1

### 1.1. Patientenproben

Von der Abteilung für Urologie, Universität Tübingen, wurden zwei Proben erhalten, die von Patienten stammten, die histologisch bestätigte Nierenzelltumore aufwiesen. Beide Patienten hatten keine präoperative Therapie erhalten. Patient Nr. 1 (im folgenden mit RCC01 bezeichnet) besaß die folgende HLA-Typisierung: HLA-A*02 A*68 B*18 B*44; der Patient Nr. 2 (im folgenden mit RCC13 bezeichnet) HLA-A*02 A*24 B*07 B*40.

### 1.2. Isolierung der MHC-Klasse-I-gebundenen Peptide

Die schockgefrorenen Tumorproben wurden prozessiert wie bereits beschrieben in Schirle, M. et al., Identification of tumor-associated MHC class I ligands by a novel T cell-independent approach, 2000, European Journal of Immunology, 30:2216-2225. Die Peptide wurden nach Standardprotokollen isoliert, und zwar unter Verwendung des monoklonalen Antikörpers W6/32, der spezifisch für die HLA-Klasse-I-Moleküle ist, oder des monoklonalen Antikörpers BB7.2, der für HLA-A2 spezifisch ist. Barnstable, C.J. et al., Production of monoclonal antibodies to group A erythrocytes, HLA and other human cell surface antigens-new tools for genetic analysis, 1978, Cell, 14:9-20 und Parham, P. & Brodsky, F.M., Partial purification and some properties of BB7.2. A cytotoxic monoclonal antibody with specificity for HLA-A2 and a variant of HLA-A28, 1981, Hum. Immunol., 3:277-299, beschrieben die Herstellung und Anwendung dieser Antikörper.

### 1.3. Massenspektroskopie

Peptide, die aus dem Tumorgewebe des Patienten RCC01 gewonnen wurden, wurden durch "reversed phase HPLC" getrennt (SMART-System, µRPC C2/C18 SC 2.1/19, Amersham Pharmacia Biotech) und die erhaltenen Fraktionen durch nanoESI MS analysiert. Dabei wurde vorgegangen, wie beschrieben in Schirle, M. et al., Identification of tumor-associated MHC class I ligands by a novel T cell-independent approach, 2000, European Journal of Immunology, 30:2216-2225.

Die Peptide, die aus Tumorgewebe des Patienten RCC13 gewonnen wurden, wurden wie eben erwähnt durch Kapillar-LC-MS identifiziert, allerdings mit geringfügigen Änderungen: 100 µl der Proben wurden jeweils geladen, entsalzt und auf einer 300 µm * 5 mm C18 p-Vorsäule (LC Packings) vorkonzentriert. Das Lösungsmittel und die Probe wurden mittels einer Spritzenpumpe (PHD 2000, Harvard Apparatur, Inc.) mit einer abgedichteten 100 µl-Spritze (1710 RNR, Hamilton) mit einer Geschwindigkeit von 2 µl/min zugeführt. Zur Trennung der Peptide wurde die Vorkonzentrierungssäule vor eine 75 µm * 250 mm C-18-Säule (LC Packings) geschaltet. Anschließend wurde ein binärer Gradient mit 25-60% B innerhalb von 70 min gefahren, wobei die Flussrate von 12 µl/min auf ungefähr 300 nl/min reduziert wurde, und zwar unter Verwendung eines TEE-Anschlusses (ZT1C, Valco) und einer 300 µm * 150 mm C-18-Säule.

Um sicherzustellen, dass das System frei von restlichen Peptiden war, wurde jeweils eine Leer-Probe gemessen. Online-Fragmentierung wurde wie beschrieben durchgeführt, und die Spektren der Fragmente wurden manuell analysiert. Die Datenbankrecherchen (NCBInr, EST) wurden unter Verwendung von MASCOT durchgeführt (http://www.matrixscience.com).

### 1.4. Identifizierung von 77 MHC-Klasse-I-Liganden aus Tumorgewebe des Patienten RCC01

In der beigefügten Tabelle 1 sind die Liganden aufgeführt, die an HLA-A*02, HLA-A*68, HLA-8*18 oder HLA-B*44 gebunden waren. Die Peptide, die mit HLA-A*02 assoziiert waren, wiesen das allel-spezifische Peptidmotiv auf: So waren an Position 2 Leucin, Valin, Isoleucin, Alanin oder Methionin und am C-Terminus Leucin, Valin, Isoleucin, oder Alanin zu finden. Die meisten der Liganden stammten von sogenannten "housekeeping"-Proteinen ab, jedoch konnten auch Liganden von Proteinen identifiziert werden, die mit Tumoren assoziiert sind.

Mit HLA-A*68 assoziierte Liganden wurden durch ihre Ankeraminosäuren Threonin, Isoleucin, Valin, Alanin oder Leucin an Position 2 und Arginin oder Lysin am C-terminalen Ende identifiziert. Dies deutete auf den Subtyp HLA-A*6801 hin. Zu den HLA-A*68-assoziierten Peptiden zählten zwei Liganden von Adipophilin, MTSALPEIQK und MAGDIYSVFR, weiterhin auch das vom Tumor-assoziierten Zyclin D1 abstammende ETIPLTAEKL. Von Annexin II stammt das Peptid TIVNILTNR ab, dieses Protein erwies sich im Zusammenhang mit MHC-Klasse-II bei Melanom-Patienten als immunogen (siehe Heinzel et al., The self peptide annexin II (208 - 223) presented by dendritic cells sentisizes autologous CD4+ T lymphocytes to recognize melanoma cells, 2001, Cancer Immunol. Immunother. 49:671-678). Die weiteren Liganden wiesen an Position 2 Glutaminsäure auf, welche eine Ankeraminosäure für den HLA-B*44-Subtyp ist. Das Peptidmotiv für HLA-B*18 ist bislang unbekannt, weshalb die Liganden dieser beiden HLA-B-Moleküle nicht unterschieden werden konnten.

### 1.5. MHC-Klasse-I-Liganden aus Tumorgewebe des Patienten RCC13

Auch für dieses Tumorgewebe konnten die gleichen Liganden identifiziert werden, die bei dem Patienten RCC01 identifiziert wurden und die von met-Protoonkogen (c-Met) und Keratin 18 abstammten. Dies waren die Peptide mit der SEQ ID-Nr. Nr. 1 und 3. Darüber hinaus konnten noch andere Liganden aus diesem Tumorgewebe gewonnen werden: So wurde bspw. ein Ligand identifiziert, der von der Nicotinamid-N-Methyltransferase (NNMT) abstammt, einem Gen, das in mehr als 95 % aller Nierentumore überexprimiert wird. Weiterhin überlappen einige weitere Liganden mit dem Peptid-Repertoir von RCC01.

### 1.6. Nachweis von Keratin 18-spezifischen T-Zellen im normalen CD8⁺-T-Zell-Repertoir

Mononucleare Zellen aus peripherem Blut gesunder Patienten wurden mit HLA-A*0201-Tetrameren gefärbt, welche entweder mit den Adipophilin-, Keratin 18- oder met-Protoonkogen (c-Met)-Peptiden konstituiert waren: Zur Herstellung der Tetramere wurden rekombinante HLA-A*0201-Moleküle *in vitro* mit den Peptiden SVASTITGV (SEQ ID-Nr. 2, Adipophilin), ALLNIKVKL (SEQ ID-Nr. 3, Keratin 18) oder YVDPVITSI (SEQ ID-Nr. 1, met-Protoonkogen, c-Met) konstituiert, durch Gelfiltration gereinigt, biotinyliert und mit Streptavidin zur Verknüpfung der Monomere gemischt.

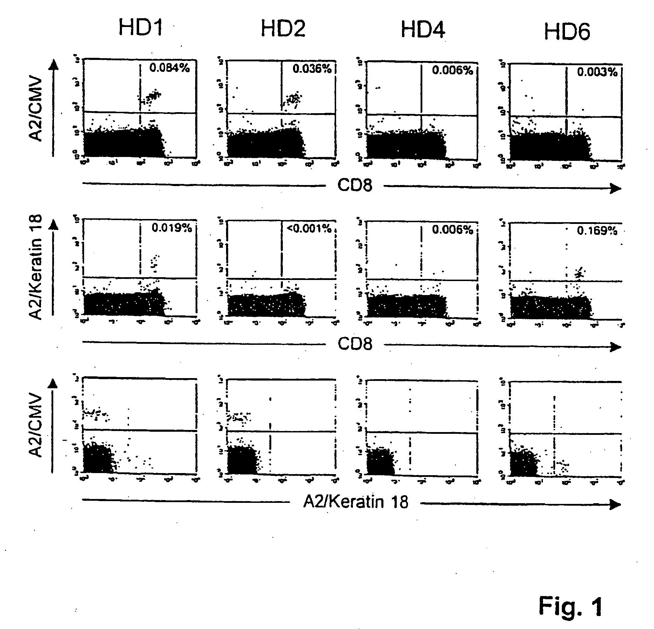

Überraschenderweise konnte eine signifikante Population von CD8⁺-T-Lymphozyten gefunden werden, die für Keratin 18 bei vier von 22 gesunden Patienten spezifisch war. In Fig. 1 sind die Ergebnisse der Doppelfärbung anhand von Dotplots gezeigt, wobei die mittlere Reihe die Ergebnisse der Färbung mit Keratin 18 zeigt. Zwischen 0,02 und 0,2 % der CD8⁺-T-Zellen war spezifisch für Keratin 18. Aus der unteren Reihe der Dotplots ist zu erkennen, dass diese Bindung des Keratin 18-Tetramers spezifisch war.

### Beispiel 2

Um die Präsentation der Peptide mit der SEQ ID-Nr. 1 (YVDPVITSI) (Peptidfragment des c-Met-Protoonkogens) und SEQ ID Nr. 2 (Peptidfragment von Adipophilin) durch Tumorzellen und die Erkennung der Peptide durch CTL zu analysieren, wurden *in vitro* CTL induziert, die spezifisch für das c-Met-Peptid (Peptid mit der SEQ ID-Nr. 1) waren und CTL, die spezifisch für das Adipophilin-Peptid (SEQ ID-Nr. 2) waren. Dazu wurden dendritische Zellen (DC) eingesetzt, die aus peripheren Blut-mononukleären-Zellen (PBMNC) von gesunden, HLA-A*02-positiven Spendern stammten.

### 2.1. Gewinnung der DC

Die DC wurden durch Ficoll/Paque-(Biochrom, Berlin, Deutschland)-Dichtegradienten-Zentrifugation aus PBMNC von heparinisiertem Blut isoliert. Das heparinisierte Blut wurde aus "buffy coat"-Präparationen gesunder Spender der Blutbank der Universität Tübingen gewonnen. Die Zellen wurden auf 6-well-Platten (Falcon, Heidelberg, Deutschland) (1 x 10⁷ Zellen/ 3 ml pro well) in RP10 Medium (RPMI 1640, ergänzt mit 10 % Hitze-inaktiviertem fetalem Kälberserum und mit Antibiotika) ausgesät. Nach einer 2-stündigen Inkubation bei 37°C und 5 % CO₂ wurden die nicht-adhärierenden Zellen entfernt und die adhärierenden Blutmonozyten in RP10 Medium kultiviert, wobei folgende Zytokine ins Medium ergänzend zugegeben wurden: humanes rekombinantes GM-CSF (granulocyte makrophage colony stimulating factor; Leukomax, Novartis; 100ng/ml), Interleukin IL-4 (R&D Systems, Wiesbaden, Deutschland; 1000 IU(ml) und TNF-α (Tumor-Nekrose-Faktor α) (R&D Systems, Wiesbaden, Deutschland; 10 ng/ml).

### 2.2. Synthese der Peptide

Beispielhaft wurden zwei HLA-A*02 bindenden Peptide (c-Met (SEQ ID-Nr. 1, YVDPVITSI) oder Adipophilin (SEQ-ID-Nr. 2, SVASTITGV), die wie oben erläutert identifiziert wurden) durch Verwendung von F-moc (9-Fluorenylmethyloxycarbonyl) - Schutzgruppen auf einem Peptid-Synthetisierer (432A, Applied Biosystems, Weiterstadt, Deutschland) synthetisiert und durch "reversed phase" HPLC und Massenspektroskopie analysiert. Auf diese Weise können ausreichende Mengen an den identifizierten Peptiden hergestellt werden.

### 2.3. Induktion einer Antigen-spezifischen CTL-Antwort unter Einsatz von HLA-A*02 restringierten synthetischen Peptiden

Zur Induktion von CTL wurden die in Schritt 2.1. gewonnenen DC (5 x 10⁵) mit den aus Schritt 2.2. erhaltenen Peptiden mit der SEQ ID-Nr. 1 oder der SEQ ID-Nr. 2 mit je 50 µg/ml für 2 Stunden gepulst, anschließend gewaschen und mit 2,5 x 10⁶ autologen PBMNC in RP10 Medium inkubiert. Nach einer 7-tägigen Kultivierungszeit wurden die Zellen mit autologen, Peptid-gepulsten PBMNC restimuliert. Dabei wurde 1 ng/ml humanes rekombinantes Interleukin IL-2 (R&D Systems) an den Tagen 1, 3 und 5 hinzugefügt. Die zytotoxische Aktivität von auf diese Weise induzierten CTL wurde an Tag 5 nach der letzten Restimulierung mittels eines standardisierten ⁵¹Cr-Freisetzungs-Assays (siehe unten, unter 2.4.: CTL-Assay) untersucht.

### 2.4. CTL-Assay

Für die CTL-Assays wurden als Target-Zellen Tumorzellen, Peptid-gepulste Zellen verschiedener Zelllinien und autologe DC verwendet. Peptid-gepulste Zellen wurden mit 50 µg/ml Peptid (SEQ ID-Nr. 1 oder SEQ ID-Nr. 2) 2 Stunden lang gepulst. Alle Target-Zellen wurden in RP10Medium (RPMI 1640, ergänzt mit 10 % Hitze-inaktiviertem fetalem Kälberserum und mit Antibiotika) 1 Stunde lang bei 37°C mit [⁵¹Cr]Natriumchromat (⁵¹Cr) markiert. Anschließend wurden jeweils 10⁴ Zellen/pro well auf eine 96-well-Platte mit abgerundetem Boden gegeben. Verschiedene Mengen an CTL wurden hinzugefügt, um ein Endvolumen von 200 µl zu erreichen, mit anschließender Inkubation für 4 Stunden bei 37°C. Danach wurden die Überstände (50µl/well) geerntet und in einem beta-Platten-Zähler ausgezählt. Die spezifische Lyse wurde in Prozent folgendermaßen berechnet: 100 x (experimentelle Freisetzung - spontane Freisetzung / maximale Freisetzung - spontane Freisetzung). Die spontane und die maximale Freisetzung wurde jeweils in Gegenwart von entweder Medium oder 2 % Triton X-100 bestimmt.

### 2.5. Ergebnisse der CTL-Induktion

### a) CTL-zytotoxische Aktivität gegenüber Peptid-gepulsten DC

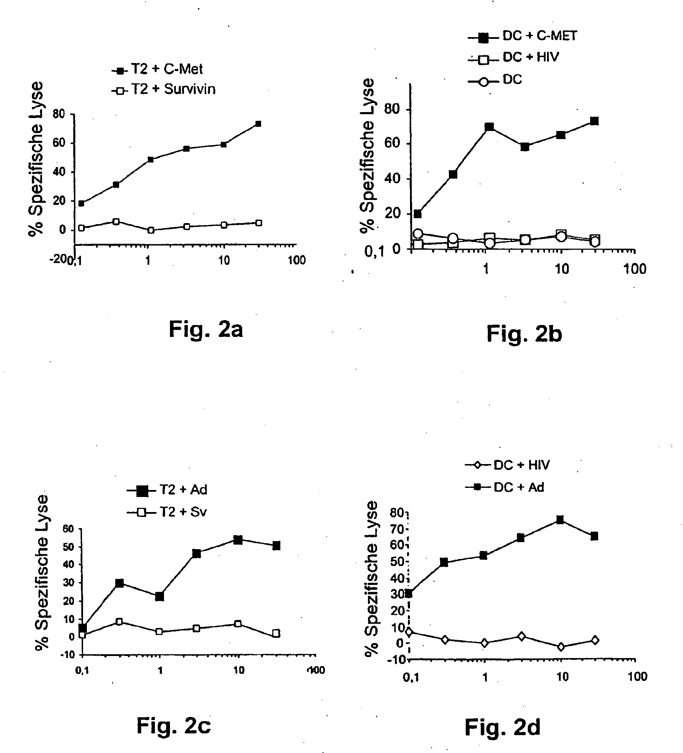

In Fig. 2 sind die Ergebnisse des ⁵¹Cr-Freisetzungs-Assays (siehe unter 2.4.) bezüglich der zytotoxischen Aktivität induzierter CTL (siehe unter 2.3.) gegenüber T2- oder DC-Zellen dargestellt. Die T2-Zelllinie ist HLA-A*02-positiv und TAP (Transporter associated with Antigen processing) -defizient; (TAP-Peptid-Transporter transportieren Peptid-Fragmente eines Proteinantigens vom Zytosol ins endoplasmatische Retikulum, wo sie mit MHC-Molekülen assoziieren.)

In den Fig. 2a und 2b ist die zytotoxische Aktivität von CTL, die unter Verwendung des Peptids mit der SEQ ID-Nr. 1 induziert wurden, gegenüber T2-Zellen und DC gezeigt, wobei beide Zellarten zuvor mit dem (c-Met-)Peptid mit der SEQ ID-Nr. 1 (schwarz gefüllte Kästchen) oder einem irrelevanten Peptid (Survivin (= "Sv"; ELTLGEFLKL; SEQ ID-Nr. 80) oder HIV (ILKEPVHGV; Pol. HIV-1 reverse Transkriptase Peptid, Aminosäurenposition 476-484; SEQ ID-Nr. 81) gepulst wurden. In Fig. 2c und 2d ist die zytotoxische Aktivität von CTL, die unter Verwendung des Peptids mit der SEQ ID-Nr. 2 induziert wurden, gegenüber T2- und DC-Zellen gezeigt, die zuvor mit dem (Adipophilin)-Peptid mit der SEQ ID-Nr. 2 gepulst wurden.

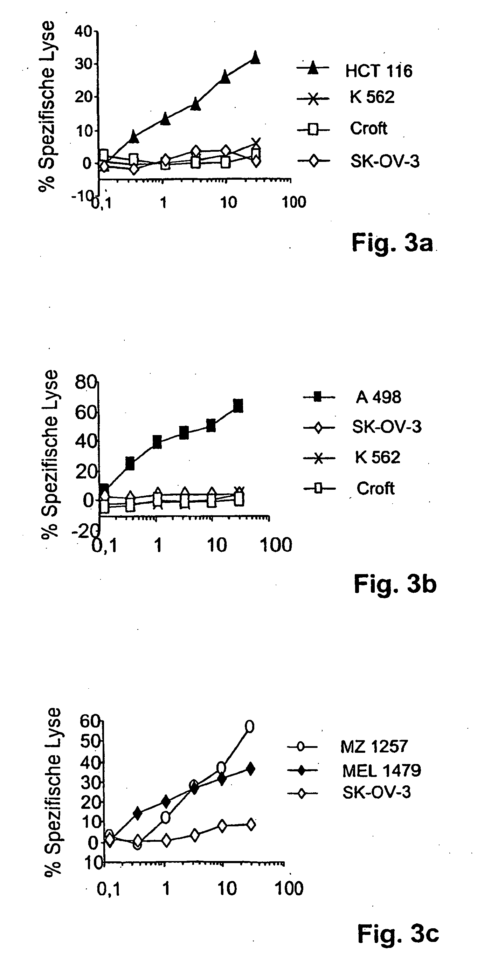

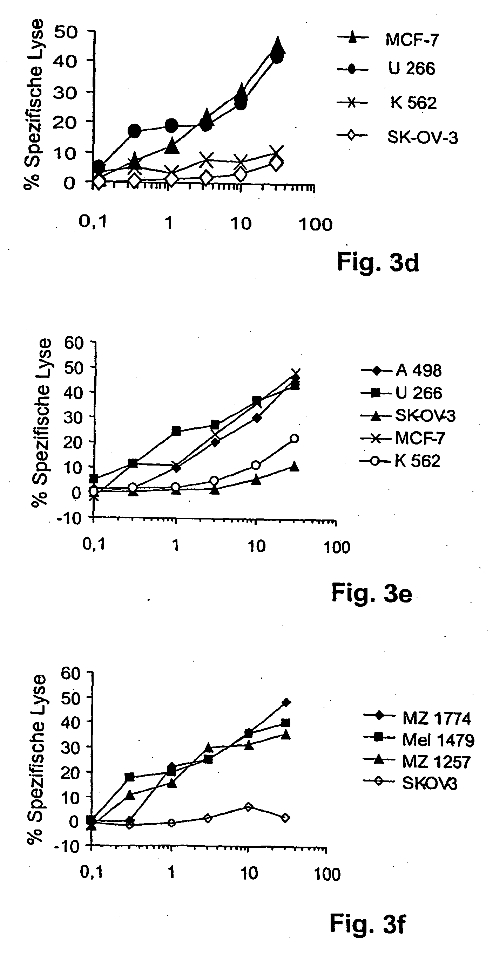

Die spezifische Lyse, die sich in der Freisetzung von ⁵¹Cr spiegelt, ist in den Fig. 2a bis 2d - wie auch in den CTL-Lysis-Diagrammen der Fig. 3 bis 5 - gegen unterschiedliche Verhältnisse von Effektorzellen (CTL) zu Zielzellen (zu lysierende, ⁵¹Cr-markierte Zellen) aufgetragen.

Wie in den Fig. 2a bis 2d zu erkennen ist, konnte mit einer CTL-Zelllinie, die nach 2-wöchiger Restimulation gewonnen wurde, eine Antigen-spezifische Abtötung der Zellen gezeigt werden: Nur diejenigen Zellen wurden durch eine ansteigende Menge an CTL abgetötet, die entweder das c-Met-Peptid mit der SEQ ID-Nr. 1 präsentierten (Fig. 2a und 2b) oder das Adipophilin-Peptid mit der SEQ ID Nr. 2 (Fig. 2c und 2d) (siehe in den Fig. 2a bis 2d jeweils die Kurven mit den schwarz ausgefüllten Kästchen-Symbolen); die mit irrelevanten Peptiden beladenen Kontrollzellen wurden nicht abgetötet (Kurven mit leeren Kästchen-Symbolen). Dadurch konnte die Spezifität der zytolytischen Aktivität gezeigt werden.

### b) CTL-zytotoxische Aktivität gegenüber Tumorzelllinien

In einem nächsten Schritt wurde wiederum anhand eines ⁵¹Cr-Freisetzungs-Assays getestet, ob die CTL, die spezifisch für das c-Met-Peptid mit der SEQ ID-Nr. 1 oder für das Adipophilin-Peptid mit der SEQ ID-Nr. 2 sind, Tumorzellen erkennen und lysieren, welche das c-Met-Protoonkogen oder Adipophilin endogen exprimieren.

Hierzu wurden die folgenden ⁵¹Cr-markierten, HLA-A*02-positiven Zelllinien eingesetzt: HCT 116 (Darmkrebs; erhalten von Prof. G. Pawelec, Tübingen, Deutschland), A 498, MZ 1257 und MZ 1774 (Nierenzellkarzinom; erhalten Prof. A. Knuth, Frankfurt, Deutschland), MCF-7 (Brustkrebs; käuflich erworben von der ATCC, American Type Culture Collection), Mel 1479 (Melanom; erhalten von Prof. G. Pawelec, Tübingen, Deutschland) und U 266 (multiples Myelom; erhalten von Prof. G. Pawelec, Tübingen, Deutschland). Diese Zelllinien exprimieren das c-Met-Protoonkogen und Adipophilin als Zielstrukturen ("targets").

Die B-Zelllinie Croft (EBV(Epstein-Barr-Virus)-immortalisiert; HLA-A*02-positiv; erhalten von O.J. Finn, Pittsburgh, USA) und die Zelllinie SK-OV-3 (Ovarialtumor; HLA-A*03-positiv; erhalten von O.J. Finn, Pittsburgh, USA) wurden als Negativkontrollen in die Studien mit aufgenommen. K 562 Zellen (bspw. erhältlich bei der Deutschen Sammlung von Mikroorganismen und Zellkulturen, DSMZ; ACC 10) wurden verwendet, um die Aktivität natürlicher Killerzellen (NK) zu bestimmen, da diese Zelllinie hoch sensitiv gegenüber diesen Killerzellen ist.

Alle Zelllinien wurden in RP10 Medium (RPMI 1640, ergänzt mit 10 % Hitze-inaktiviertem fetalem Kälberserum und mit Antibiotika) kultiviert.

Mit den o.g. Tumor-Zelllinien und der wie unter 2.3. induzierten CTL wurden ⁵¹Cr-Freisetzungsassays (siehe unter 2.4.) durchgeführt.

Die Figuren 3a bis 3f zeigen die Ergebnisse dieser CTL-Assays, wobei in den Fig. 3a bis 3d CTL eingesetzt wurden, die unter Verwendung des c-Met-Peptids mit der SEQ ID-Nr. 1 induziert wurden, und in den Fig. 3e und 3f CTL, die unter Verwendung des Adipophilin-Peptids mit der SEQ ID-Nr. 2 induziert wurden.

Wie in den Figuren 3a bis 3f zu erkennen ist, lysierten die CTL, die für das c-Met-Peptid mit der SEQ ID-Nr. 1 (Fig. 3a bis 3d) oder für das Adipophilin-Peptid mit der SEQ ID-Nr. 2 (Fig. 3e und 3f) spezifisch sind, effizient Tumorzellen, die sowohl HLA-A*02 als auch c-Met oder Adipophilin exprimieren (also in Fig. 3a die Zelllinie HCT 116, in Fig. 3b die Zelllinie A 498, in Fig. 3c die Zelllinien MZ 1257 und MEL 1479 und in Fig. 3d die Zelllinien MCF-7 und U 266; in Fig. 3e die Zelllinien A 498, U 266 und MCF-7, in Fig. 3f die Zelllinien MZ 1774, Mel 1479 und MZ 1257. Die spezifische Lyse wurde - wie oben unter 2.4. angegeben - durch die ⁵¹Cr-Freisetzung gemessen. Die Kontroll-Zelllinie SK-OV-3 (HLA-A-*02-negativ) hingegen wurde weder durch CTL lysiert, die durch das Peptid mit der SEQ ID-Nr. 1 induziert wurden, noch durch CTL, die durch das Peptid mit der SEQ ID-Nr. 2 induziert wurden. Dies zeigte, dass beide Peptide im Zusammenhang mit HLA-A*02-Molekülen auf den Tumorzellen präsentiert werden müssen, um die Target-Zellen effizient zu lysieren. Ferner wird dadurch die Antigen-Spezifität und die MHC-Restriktion der CTL bestätigt.

Die *in vitro* durch das Peptid mit der SEQ ID-Nr. 1 induzierten CTL-Zellen erkannten darüber hinaus auch nicht die Zelllinie K562 (siehe Fig. 3a, 3b und 3d), was zeigt, dass die zytotoxische Aktivität nicht durch Natürliche Killerzellen (NK)-Zellen vermittelt wurde.

### c) Inhibitions-Assays

Um die Antigen-Spezifität und die MHC-Restriktion der *in-*vitro-induzierten CTL weiter zu verifizieren, wurden Inhibitions-Assays mit nicht-⁵¹Cr-markierten ("kalten") Inhibitor-Zelllinien durchgeführt.

Hierbei wurde die Fähigkeit von Peptid-gepulsten Zelllinien analysiert, die Lyse von Tumor-Zellen zu inhibieren, bzw. zu kompetitieren. Dazu wurde ein Überschuss an Inhibitor (also an gepulsten, unmarkierten Zellen) eingesetzt. Das Verhältnis des Inhibitors (Peptid-gepulste Zellen) zum Target (Tumor-Zellen) betrug 20:1. Bei Lyse der Inhibitor-Zelllinien konnte kein ⁵¹Cr freigesetzt werden, da die Inhibitor-Zelllinien unmarkiert waren.

Als Inhibitor wurde die Zelllinie T2 (HLA-A*02; TAP-defizient; siehe unter 2.5.a)) eingesetzt. Diese Zelllinie T2 wurde vor den Assays jeweils mit den relevanten Peptiden (SEQ ID-Nr. 1 oder 2) oder einem irrelevanten Kontrollpeptid (Survivin (=Sv), SEQ ID-Nr. 80) gepulst.

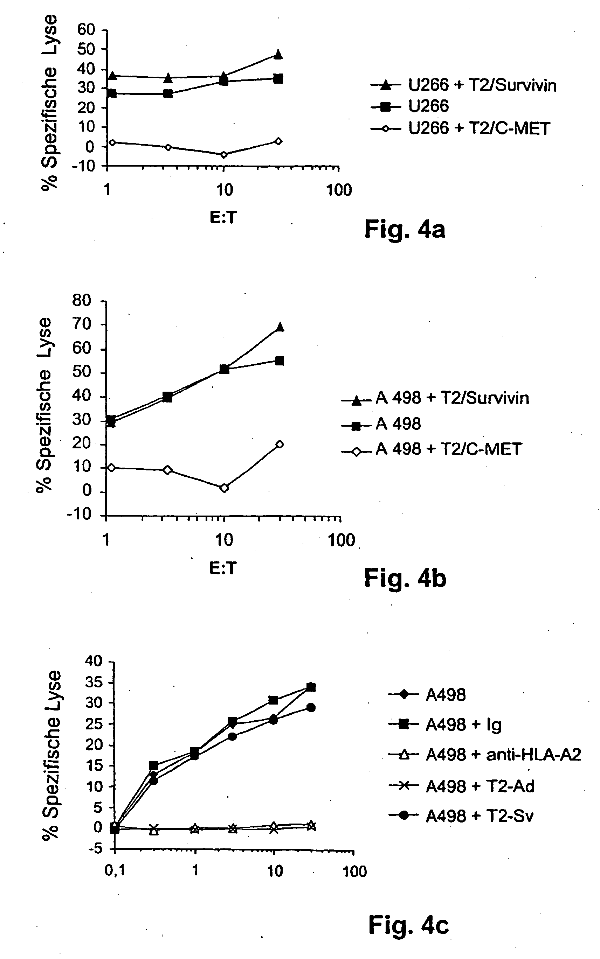

Die Ergebnisse dieser Tests sind in den Fig. 4a bis 4c gezeigt, wobei in den Fig. 4a und 4b CTL eingesetzt wurden, die durch das c-Met-Peptid mit der SEQ ID-Nr.1 induziert wurden und in Fig. 4c CTL, die durch das Adipophilin-Peptid mit der SEQ ID-Nr. 2 induziert wurden.

In Fig. 4a und 4b wurde die Lyse der ⁵¹Cr-markierten Zelllinien U 266 und A 498 getestet, und zwar ohne Inhibitor-Zelllinie (jeweils die Kurve mit den schwarz ausgefüllten Kästchen); mit der Inhibitor-Zelllinie T2, gepulst mit einem irrelevanten Peptid (Survivin; SEQ ID-Nr. 80; Negativkontrolle, Kurven mit den ausgefüllten Dreiecken); und mit der Inhibitor-Zelllinie T2, gepulst mit dem c-Met-Peptid mit der SEQ ID-Nr. 1 (Kurven mit den nicht ausgefüllte Rhomben).

Ohne Vorliegen der Inhibitor-Zellen wurde eine Lyse der Tumorzellen durch CTL beobachtet (siehe in den Fig. 4a bis 4d jeweils die Kurven mit den schwarz ausgefüllten Kästchen). Wie den Abbildungen 4a und 4b ferner zu entnehmen ist, fand bei einem Überschuss an Inhibitor-Target keine Lyse der Tumorzellen statt (und somit keine ⁵¹Cr-Freisetzung), sofern das Inhibitor-Target mit dem c-Met-Peptid mit der SEQ ID-Nr. 1 gepulst war (siehe jeweils die Kurven mit den nicht ausgefüllten Rhomben-Symbolen). Die Aktivität der CTL richtete sich auf die im Überschuss vorliegenden, unmarkierten T2-Zellen, so dass diese und nicht die Tumorzellen lysiert wurden. Die T2-Zellen, die mit einem irrelevanten Peptid (jeweils Survivin; SEQ ID-Nr. 80) gepulst waren, konnten die Lyse der Tumorzellen durch die CTL nicht inhibieren, so dass freigesetztes sich gemessen werden konnte (siehe in Fig. 4a und 4b die Kurven mit den schwarz ausgefüllten Dreiecken).

Ähnliches konnte bei Verwendung von CTL beobachtet werden, die durch Einsatz des Adipophilin-Peptids mit der SEQ ID-Nr. 2 induziert wurden (siehe Fig. 4c):

Die MHC-Restriktion und die Antigen-Spezifität der zytotoxischen Aktivität, die durch die Adipophilin-induzierten CTL vermittelt wurde, konnte unter Verwendung eines HLA-A*02-spezifischen monoklonalen Antikörpers und in einem Inhibitions-Assay mit unmarkiertem ("kaltem") Inhibitor bestätigt werden: Die Ergebnisse dieses Versuchs sind in Fig. 4c gezeigt. Die A 498-Tumorzellen wurden durch Zugabe des HLA-A*02-spezifischen Antikörpers (monoklonaler Antikörper BB7.2, IgG2b, erhalten von S. Stefanovic, Tübingen) blockiert, so dass sie durch Zugabe der CTL nicht lysiert wurden und kein ⁵¹Cr freigesetzt wurde (siehe Fig. 4c die Kurve mit nicht ausgefüllten Dreiecks-Symbolen). Als Kontrolle diente ein unspezifischer Antikörper, der das HLA-A*02-Molekül nicht blockierte (ChromPure Maus IgG, Dianova, Deutschland; siehe in Fig. 4c die Kurve mit den ausgefüllten Kästchen). Für diese Inhibierungs-Versuche wurden die Zellen vor Aussaat auf den 96-well-Platten 30 min. mit 10 µg/ml Antikörper inkubiert.

Ferner zeigte sich, dass die T2-Kompetitions-Zelllinie, die mit dem irrelevanten Peptid Survivin mit der SEQ ID-Nr. 80 gepulst wurde (T2/SV), die CTL-vermittelte Lyse der Tumor-Zelllinie A 498 nicht inhibieren konnte (siehe in Fig. 4c die Kurve mit schwarz ausgefüllten Kreisen), dass jedoch die mit dem Adipophilin-Peptid mit der SEQ ID-Nr. 2 gepulste T2-Inhibitor-Zelllinie (T2/AD) die Lyse der Tumor-Zelllinie inhibieren konnte, so dass in letzterem Fall keine ⁵¹Cr-Freisetzung gemessen werden konnte (siehe in Fig. 4c die Kurve mit x-Symbolen).

### d) Spezifische Lyse transfizierter DC

In einem nächsten Versuch wurde die zytotoxische Aktivität der CTL bei einem autologen Versuchsansatz analysiert. Hierzu wurden autologe DC, die aus den gleichen PBMNC gewonnen wurden wie diejenigen, die für die CTL-Induktion (siehe unter 2.2.) verwendet wurden, als Target-Zellen eingesetzt. Vor Durchführung des CTL-Assays wurden die DC mit RNA elektroporiert, welche zuvor entweder aus Tumor-Zelllinien isoliert wurde oder welche Kontroll-RNA darstellte (*in vitro* transkribierte EGFP-RNA (enhanced Green fluorescent Protein-RNA); verwendetes Plasmid: pSP64 Poly(A) EGFPII, erhalten von Van Tendeloo, Antwerpen, Belgien). Die Gesamt-RNA wurde aus den Tumorzellen mittels des QIAGEN Rneasy Mini Kits (QIAGEN, Hilden, Deutschland) nach Angaben des Herstellers isoliert.

Menge und Reinheit der RNA wurde photometrisch bestimmt und in Aliquots bei -80°C aufbewahrt.

Vor der Elektroporation an Tag 6 wurden unreife DC zweimal mit Serum-freiem X-VIVO 20 Medium (BioWhittaker, Walkersville, USA) gewaschen und in einer Endkonzentration von 2 x 10⁷ Zellen/ml resuspendiert. Anschließend wurden 200 µl der Zellsuspension mit 10 µg der Gesamt-RNA gemischt und in einer 4 mm Küvette mittels eines Easyject Plus^{™} (Peqlab, Erlangen Deutschland) elektroporiert (Parameter: 300 V, 150 µF, 1540 Ω, Pulszeit: 231 ms). Nach der Elektroporation wurden die Zellen sofort in RP10 Medium überführt und wieder in den Inkubator gegeben. Mehr als 80 % der Zellen waren nach der Elektroporation lebensfähig.

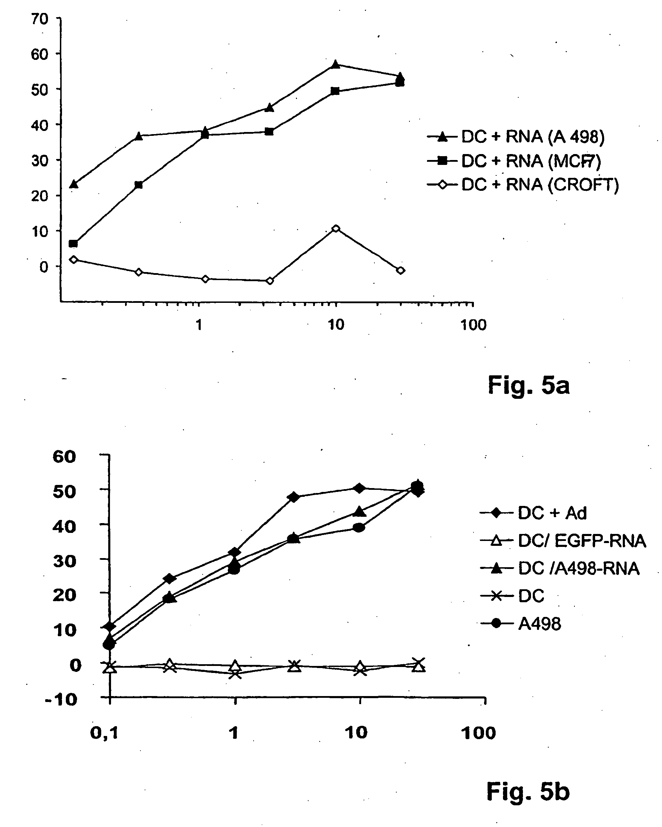

Die Ergebnisse dieser Versuchsansätze sind in den Fig. 5a und 5b gezeigt. In Fig. 5a wurden CTL eingesetzt, die durch Verwendung des c-Met-Peptids mit der SEQ ID-Nr. 1 induziert waren, in Fig. 5b wurden CTL eingesetzt, die durch Verwendung des Adipophilin-Peptids mit der SEQ ID-Nr. 2 induziert waren.

Nach Durchführung des CTL-Assays mit durch das c-Met-Peptid mit der SEQ ID-Nr. 1 induzierten CTL (siehe unter 2.4.) konnte eine spezifische Lyse von DC beobachtet werden, welche mit RNA von c-Met-exprimierenden Tumor-Zelllinien (A 498 und MCF-7) elektroporiert waren (siehe in Fig. 5a die Kurven mit den schwarz ausgefüllten Symbolen). DC, die mit RNA der nicht c-Met-exprimierenden Tumor-Zelllinie Croft elektroporiert waren, wurden hingegen nicht lysiert (siehe Kurve mit den nicht ausgefüllten Rhomben).

CTL, die durch das Adipophilin-Peptid mit der SEQ ID-Nr. 2 induziert waren, lysierten DC, welche mit RNA der Adipophilin-exprimierenden Zelllinie A 498 elektroporiert waren (siehe in Fig. 5b die Kurve mit den schwarz ausgefüllten Dreiecken). Ferner wurden DC lysiert, die mit dem Adipophilin-Peptid mit der SEQ ID-Nr. 2 gepulst waren (siehe in Fig. 5b die Kurve mit den schwarz ausgefüllten Rhomben). Hingegen wurden DC, die mit Kontroll-(EGFP)RNA elektroporiert wurden, nicht lysiert (siehe in Fig. 5b die Kurve mit den nicht ausgefüllten Dreiecken).

Dies zeigt, dass - nach Transfektion der DC mit RNA von c-Met- oder Adipophilin-positiven Tumor-Zellen - die identifizierten Peptide, also das c-Met-Peptid mit der SEQ ID-Nr. 1 und das Adipophilin-Peptid mit der SEQ ID-Nr. 2, prozessiert und präsentiert werden.

### e) Induktion von Adipophilin-spezifischen CTL in einem Patienten mit chronisch lymphatischer Leukämie

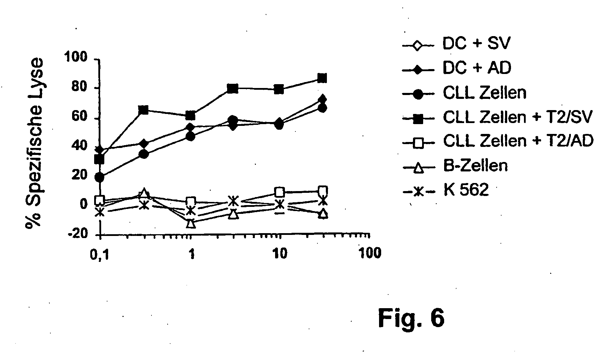

In einem weiteren Versuch wurden aus PBMNC eines HLA-A-*0201-positiven Patienten mit chronisch lymphatischer Leukämie (CLL) CTL generiert, die für das Adipophilin-Peptid mit der SEQ ID-Nr.2 spezifisch waren. Der Patient befand sich nach einer Behandlung mit Fludarabin in Remission. Ferner wurden autologe primäre CLL-Zellen und DC dieses Patienten als ⁵¹Cr-markierte Targets in einem Assay eingesetzt, bei welchem eine ⁵¹Cr-Freisetzung durch die Peptid-induzierten CTL vermittelt wird. Wie in Fig. 6 gezeigt ist, wurden sowohl die autologen DC dieses Patienten, welche mit dem Adipophilin-Peptid mit der SEQ ID-Nr. 2 gepulst wurden ("DC+AD"), als auch die autologen CLL-Zellen ("CLL Zellen") durch die Peptid-induzierten CTL lysiert. DC, welche mit dem irrelevanten Peptid Survivin mit der SEQ ID-Nr. 80 gepulst wurden, wurden hingegen nicht lysiert ("DC+SV"). Auch nichtmaligne B-Zellen und die Zelllinie K 562 wurden nicht durch die CTL lysiert.

Die Spezifität der CTL-Antwort wurde in einem Target-Inhibitions-Assay bestätigt, wobei als Inhibitor-Zellen die Zelllinie T2 (s.o.) eingesetzt wurde, welche jeweils mit dem Adipophilin-Peptid mit der SEQ ID-Nr. 2 oder mit dem irrelevanten Peptid Survivin mit der SEQ ID-Nr. 80 gepulst waren. Die durch Verwendung des Adipophilin-Peptids mit der SEQ ID-Nr. 2 induzierten CTL lysierten auch hier die im Überschuss vorliegenden Inhibitor-Zelllinien, die mit dem relevanten Peptid mit der SEQ ID-Nr. 2 gepulst waren, so dass die ⁵¹Cr-markierten Tumorzellen in diesem Fall nicht lysiert wurden (siehe in Fig. 6 die Kurve mit den nicht ausgefüllten Kästchen).

Zusammengefasst konnten die Erfinder somit zeigen, dass die identifizierten Peptide vielversprechende Substanzen im Rahmen einer Immuntherapie bei einer Vielzahl von (Tumor-) Erkrankungen darstellen.

**Tabelle 1**

| | **Sequenz** | **Position/Genart** | **Acc. Nr.** | **SEQ ID-Nr.** | |
|---|---|---|---|---|---|
| **Patient RCC01** | | | | | |
| **HLA-A*02** | | | | | |
| 1. | YVDPVITSI | 654-662 | J02958 | SEQ ID-Nr. | 1 |
| | | met proto-oncogen | | | |
| 2. | SVASTITGV | 129-137 | X97324 | SEQ ID-Nr. | 2 |
| | | adipose differentiation-related protein | | | |
| 3. | ALLNIKVKL | 365-373 | M26326 | SEQ ID-Nr. | 3 |
| | | keratin 18 | | | |
| 4. | ALFDGDPHL | 1-9 | AB002365 | SEQ ID-Nr. | 4 |
| | | KIAA0367 | | | |
| 5. | RLLDYVVNI | 679-687 | AB040951 | SEQ ID-Nr. | 5 |
| | | hypothetical protein FLJ20004 | | | |
| 6. | ALANGIEEV | 101-109 | AY014906 | SEQ ID-Nr. | 6 |
| | | apolipoprotein L, 3 | | | |
| 7. | QLIDKVWQL | 593-601 | D67029 | SEQ ID-Nr. | 7 |
| | | SEC14 (S.cerevisiae)-like 1 | | | |
| 8. | ALSDLEITL | 389-397 | Z24725 | SEQ ID-Nr. | 8 |
| | | mitogen inducible 2 | | | |
| 9. | ILDTGTIQL | 174-182 | AB013094 | SEQ ID-Nr. | 9 |
| | | kidney- and liver-specific gene | | | |
| 10. | SLLGGDVVS | 27-36 | AF153603 | SEQ ID-Nr. | 10 |
| | V | delta sleep inducing peptide, immunoreactor | | | |
| 11. | FLDGNELTL | 167-175 | U93205 | SEQ ID-Nr. | 11 |
| | | chloride intracellular channel 1 | | | |
| 12. | NLLPKLHIV | 179-187 | U93205 | SEQ ID-Nr. | 12 |
| | | chloride intracellular channel 1 | | | |
| 13. | ALASHLIEA | 507-515 | AF181263 | SEQ ID-Nr. | 13 |
| | | EH-domain containing 2 | | | |
| 14. | SLYGGTITI | 296-304 | AK000697 | SEQ ID-Nr. | 14 |
| | | hypothetical protein FLJ 11189 | | | |
| 15. | FLLDKKIGV | 218-226 | AF026166 | SEQ ID-Nr. | 15 |
| | | chaperonin containing TCPI, subunit 2 (beta) | | | |
| 16. | FLDGNEMTL | 178-186 | AF097330 | SEQ ID-Nr. | 16 |
| | | chloride intracellular channel 4 | | | |
| 17. | AIVDKVPSV | 147-155 | AF100756 | SEQ ID-Nr. | 17 |
| | | coat-protein gamma-cop | | | |
| 18. | DVASVIVTK | 241-250 | U51920 | SEQ ID-Nr. | 18 |
| | L | signal recognition particle 54kD | | | |
| 19. | LASVSTVL | 130-137 | AF230076 | SEQ ID-Nr. | 19 |
| | | hemoglobin, alpha 2 | | | |
| 20. | VMAPRTLVL | 3-11 | | SEQ ID-Nr. | 20 |
| | | HLA-A | | | |
| 21. | LLFDRPMHV | 267-275 | L03532 | SEQ ID-Nr. | 21 |
| | | hnRNP M | | | |

| **HLA-A*68** | | | | | |
|---|---|---|---|---|---|
| 22. | MTSALPIIQK | 62-71 | X97324 | SEQ ID-Nr. | 22 |
| | | adipose differentiation-related protein | | | |
| 23. | MAGDIYSVF | 349-358 | X97324 | SEQ ID-Nr. | 23 |
| | R | adipose differentiation-related protein | | | |
| 24. | ETIPLTAEKL | 115-124 | X59798 | SEQ ID-Nr. | 24 |
| | | cyclin D1/PRAD1 | | | |
| 25. | DVMVGPFKL | 934-943 | AJ303079 | SEQ ID-Nr. | 25 |
| | R | A kinase (PRKA) anchor protein 2 | | | |
| 26. | TIIDILTKR | 64-72 | X05908 | SEQ ID-Nr. | 26 |
| | | annexin A1 | | | |
| 27. | TIVNILTNR | 55-63 | BC001388 | SEQ ID-Nr. | 27 |
| | | annexin A2 | | | |
| 28. | TIIDIITHR | 385-393 | J03578 | SEQ ID-Nr. | 28 |
| | | annexin A6 | | | |
| 29. | SIFDGRVVA | 107-116 | AB020980 | SEQ ID-Nr. | 29 |
| | K | putative membrane protein | | | |
| 30. | STIEYVIQR | 115-123 | BC005032 | SEQ ID-Nr. | 30 |
| | | Sec23 (S. cerevisiae) homolog B | | | |
| 31. | ELIKPPTILR | 132-141 | AF092092 | SEQ ID-Nr. | 31 |
| | | adaptor-related protein complex3 | | | |
| 32 | EIAMATVTALR | 248-258 | X12447 | SEQ ID-Nr. | 32 |
| | | aldolase A, fructose-biphosphate | | | |
| 33. | ETIGEILKK | 95-103 | BC000355 | SEQ ID-Nr. | 33 |
| | | hnRNP K | | | |
| 34. | SLADIMAKR | 86-94 | BC000690 | SEQ ID-Nr. | 34 |
| | | ribosomal protein L24 | | | |

| **HLA-B*44 oder HLA-B*18** | | | | | |
|---|---|---|---|---|---|
| 35. | EEIAFLKKL | 229-237 vimentin | M14144 | SEQ ID-Nr. | 35 |
| 36. | DEAAFLERL | 92-100 | M64110 | SEQ ID-Nr. | 36 |
| | | caldesmon 1 | | | |
| 37. | DEMKVLVL | 545-552 | M96803 | SEQ ID-Nr. | 37 |
| | | spectrin, beta, non-erythrocytic 1 | | | |
| 38. | DEVKFLTV | 191-198 | M82809 | SEQ ID-Nr. | 38 |
| | | annexin A4 | | | |
| 39. | NENSLFKSL | 935-943 | D21260 | SEQ ID-Nr. | 39 |
| | | clathrin, heavy polypeptide (Hc) | | | |
| 40. | DEFKVVVV | 373-380 | AF100756 | SEQ ID-Nr. | 40 |
| | | coat protein, gamma-cop | | | |
| 41. | EEVKLIKKM | 137-145 | M11147 | SEQ ID-Nr. | 41 |
| | | ferritin, light polypeptide | | | |
| 42 | DEVKLPAKL | 158-166 | AF312393 | SEQ ID-Nr. | 42 |
| | | polymerase 1 and transcript release factor | | | |
| 43. | TERELKVAY | 637-645 | AB040951 | SEQ ID-Nr. | 43 |
| | | hypothetical protein FLJ20004 | | | |
| 44. | NEFSLKGVD | 86-95 | J04101 | SEQ ID-Nr. | 44 |
| | F | ets-1 | | | |
| 45. | NEQDLGIQY | 169-177 | D13866 | SEQ ID-Nr. | 45 |
| | | catenin alpha 1 | | | |
| 46. | EERIVELF | 306-313 | BC000627 | SEQ ID-Nr. | 46 |
| | | signal transducer and activator of transcription | | | |
| | | 3 | | | |
| 47. | EEIREAFRVF | 84-93 | J04046 | SEQ ID-Nr. | 47 |
| | | calmodulin 3 | | | |
| 48. | DEYIYRHFF | 344-352 | AF011794 | SEQ ID-Nr. | 48 |
| | | cell cycle progression 8 protein | | | |
| 49. | DELELHQRF | 308-316 | X86098 | SEQ ID-Nr. | 49 |
| | | adenovirus 5 E1 A binding protein | | | |
| 50. | SEVKFTVTF | 80-88 | M87842 | SEQ ID-Nr. | 50 |
| | | galectin 2 | | | |
| 51. | IETIINTF | 12-19 | M26311 | SEQ ID-Nr. | 51 |
| | | calgranulin B | | | |
| 52. | KENPLQFKF | 61-69/72-80 | J05021/ | SEQ ID-Nr. | 52 |
| | | villin 2 (ezrin)/(radixin) | L02320 | | |
| 53. | DEVRTLTY | 41-48 | Y10807 | SEQ ID-Nr. | 53 |
| | | hnRNP methyltransferase, S. cerevisiae-like 2 | | | |
| 54. | GEAVVNRVF | 43-51 | Z14977 | SEQ ID-Nr. | 54 |
| | | large multifunctional protease 2, LMP2 | | | |
| 55. | EEVLIPDQKY | 385-394 | AF126028 | SEQ ID-Nr. | 55 |
| | | F-box and leucine-rich repeat protein 3A | | | |
| 56. | DEGRLVLEF | 163-171 | L21934 | SEQ ID-Nr. | 56 |
| | | sterol O-acyltransferase 1 | | | |
| 57. | DEVELIHF | 838-845 | AF152961 | SEQ ID-Nr. | 57 |
| | | chromatin-specific transcription elongation | | | |
| | | factor | | | |
| 58. | VEVLLNYAY | 83-91 | AF205218 | SEQ ID-Nr. | 58 |
| | | NS1-binding protein | | | |
| 59. | TENDIRVMF | 120-128 | AF267534 | SEQ ID-Nr. | 59 |
| | | CUG triplet repeat, RNA-binding protein 1 | | | |
| 60. | LEGLTVVY | 62-69 | AF151878 | SEQ ID-Nr. | 60 |
| | | coatomer protein complex subunit zeta 1 | | | |
| 61. | NELPTVAF | 192-199 | AK001475 | SEQ ID-Nr. | 61 |
| | | hypothetical protein | | | |
| 62. | EEFGQAFSF | 77-85 | X03100 | SEQ ID-Nr. | 62 |
| | | MHC, class II, DP alpha I | | | |
| 63. | VEAIFSKY | 33-40 | M29063 | SEQ ID-Nr. | 63 |
| | | hnRNP C (C1/C2) | | | |
| 64. | DERTFHIFY | 277-285 | M69181 | SEQ ID-Nr. | 64 |
| | | myosin, heavy polypeptide 10, non-muscle | | | |
| 65. | TEKVLAAVY | 206-214 | K01177 | SEQ ID-Nr. | 65 |
| | | aldolase B, fructose-bisphosphate | | | |
| 66. | VESPLSVSF | 159-167 | AK025971 | SEQ ID-Nr. | 66 |
| | | hypothetical protein FLJ22318 | | | |
| 67. | SEAGSHTLQW | MHC-1 | | SEQ ID-Nr. | 67 |
| DEGKVIRF | DEGKVIRF | 56-63 | BF431469 | SEQ ID-Nr. | 68 |
| | | EST reading frame-1 | | | |

| **Patient RCC13** | | | | | |
|---|---|---|---|---|---|
| **HLA-A*02** | | | | | |
| 69. | ALAAVVTEV | frameshift, DDX3 reading frame +2 | AF061337 | SEQ ID-Nr. | 69 |
| 70. | TLIEDILGV | 209-217 | AL132825 | SEQ ID-Nr. | 70 |
| | | transient receptor protein 4 associated protein | | | |
| 71. | ALFGALFLA | 2-10 | L26232 | SEQ ID-Nr. | 71 |
| | | phospholipid transfer protein | | | |
| 72. | VLATLVLLL | 72-80 | AA483794 | SEQ ID-Nr. | 72 |
| | | EST | | | |
| 73. | TLDDLIAAV | 325-333 | AK000904 | SEQ ID-Nr. | 73 |
| | | hypothetical protein FLJ 10042 | | | |
| 74. | YLDNGVVFV | 316-324 | U18299 | SEQ ID-Nr. | 74 |
| | | damage-specific DNA binding protein 1 | | | |
| | | (127kD) | | | |
| 75. | SVFAGVVGV | 581-589 | U58855 | SEQ ID-Nr. | 75 |
| | | guanylate cyclase I, soluble, alpha 3 | | | |
| 76. | SLINVGL1SV | 48-57 | BC000476 | SEQ ID-Nr. | 76 |
| | | acidic protein rich in leucines | | | |
| 77. | ALADGVQK | 176-184 | AF323540 | SEQ ID-Nr. | 77 |
| | V | apolipoprotein L, 1) | | | |

| **HLA-A*24** | | | | | |
|---|---|---|---|---|---|
| 78. | TYGEIFEKF | 107-115 | AF070652 | SEQ ID-Nr. | 78 |
| | | NADH dehydrogenase (ubiquinone) 1, | | | |
| | | (B14.5b) | | | |
| 79. | YYMIGEQKF | 203-211 | U08021 | SEQ ID-Nr. | 79 |
| | | nicotinamide-n-methyltransferase | | | |

## Patentansprüche

1. Tumor-assoziiertes Peptid mit der Aminosäuresequenz ALFDGDPHL (SEQ ID No. 4), wobei das Peptid die Fähigkeit aufweist, an ein Molekül des menschlichen Haupt-Histokompatibilitäts-Komplexes (MHC) Klasse-I zu binden.

2. Peptid nach Anspruch 1, **dadurch gekennzeichnet, dass** eine Aminosäure durch eine andere Aminosäure mit ähnlichen chemischen Eigenschaften ersetzt ist.

3. Peptid nach Anspruch 1 oder 2, **dadurch gekennzeichnet, dass** N- oder/und C-terminal eine weitere Aminosäure vorhanden ist.

4. Peptid nach einem der Ansprüche 1 bis 3, **dadurch gekennzeichnet, dass** eine Aminosäure deletiert ist.

5. Peptid nach einem der Ansprüche 1 bis 4, **dadurch gekennzeichnet, dass** zumindest eine Aminosäure chemisch modifiziert ist.

6. Verwendung des Peptids nach einem der Ansprüche 1 bis 5 zur Herstellung eines Arzneimittels zur Behandlung von Tumorerkrankungen und/oder adenomatöser Erkrankungen.

7. Verwendung des Peptids nach einem der Ansprüche 1 bis 5 zur Herstellung einer pharmazeutischen Zusammensetzung zur Behandlung von Tumorerkrankungen und/oder adenomatöser Erkrankungen.

8. Verwendung nach Anspruch 6 oder 7, **dadurch gekennzeichnet, dass** die Erkrankung Nieren-, Brust-, Pankreas-, Magen-, Blasen- und/oder Hodenkrebs ist.

9. Verwendung nach Anspruch 7 oder 8, **dadurch gekennzeichnet, dass** das Peptid zusammen mit einem Adjuvans eingesetzt wird.

10. Verwendung nach Anspruch 7 oder 8, **dadurch gekennzeichnet, dass** das Peptid gebunden auf einer Antigen-präsentierenden Zelle eingesetzt wird.

11. Verwendung des Peptids nach einem der Ansprüche 1 bis 5 zur Herstellung eines Antikörpers.

12. Pharmazeutische Zusammensetzung enthaltend das Peptid nach einem der Ansprüche 1 bis 5.

13. Nukleinsäuremolekül, kodierend für das Peptid nach einem der Ansprüche 1 bis 5.

14. Vektor umfassend das Nukleinsäuremolekül nach Anspruch 13.

15. Zelle, die mit Hilfe des Nukleinsäuremoleküls nach Anspruch 13 oder mit Hilfe des Vektors nach Anspruch 14 genetisch derart verändert wurde, dass sie ein Peptid nach einem der Ansprüche 1 bis 5 produziert.

## Claims

1. A tumor-associated peptide comprising the amino acid sequence ALFDGDPHL (SEQ ID No. 4), the peptide having the ability to bind to a molecule of the human major histocompatibility complex (MHC) class-I.

2. Peptide according to claim 1, wherein one amino acid is replaced by another amino acid having similar chemical features.

3. The peptide according to claim 1 or 2, wherein said peptide comprises one additional amino acid at the N- or/and C-terminus.

4. The peptide according to any of claims 1 to 3, wherein one amino acid is deleted.

5. The peptide according to any of claims 1 to 4, wherein at least one amino acid is chemically modified.

6. The use of the peptide according to any of claims 1 to 5 for the production of a medicament for the treatment of tumor diseases and/or adenomatous diseases.

7. The use of the peptide according to any of claims 1 to 5 for the production of a pharmaceutical composition for the treatment of tumor diseases and/or adenomatous diseases.

8. The use according to claim 6 or 7, wherein the disease is renal, breast, pancreatic, stomach, bladder and/or testicular cancer.

9. The use according to claim 7 or 8, wherein the peptide is used together with an adjuvant.

10. The use according to claim 7 or 8, wherein the peptide being used bound to an antigen presenting cell.

11. The use of the peptide according to any of claims 1 to 5 for production of an antibody.

12. A pharmaceutical composition containing the peptide according to any of claims 1 to 5.

13. A nucleic acid molecule encoding the peptide according to any of claims 1 to 5.

14. A vector, comprising the nucleic acid molecule according to claim 13.

15. A cell that has been genetically altered using the nucleic acid molecule according to claim 13 or using the vector according to claim 14, to produce a peptide according to one of the claims 1 to 5.

## Revendications

1. Un peptide tumoral avec séquence d'acide aminé ALFDGDPHL (SEQ ID No. 4), le peptide étant capable de se fixer sur une molécule de classe I du complexe majeur d'histocompatibilité (CMH) humain.

2. Le peptide selon la revendication 1, **se caractérisant par** le remplacement d'un acide aminé par un autre acide aminé aux propriétés chimiques semblables.

3. Le peptide selon la revendication 1 ou 2, **se caractérisant par** la présence d'un acide aminé supplémentaire à l'extrémité N et/ou C.

4. Le peptide selon une des revendications 1 à 3, **se caractérisant par** la délétion d'un acide aminé.

5. Le peptide selon une des revendications 1 à 4, **se caractérisant par** la modification chimique d'un acide aminé au moins.

6. L'utilisation du peptide selon une des revendications 1 à 5 pour la fabrication d'un médicament destiné au traitement de maladies tumorales et/ou adénomateuses.

7. L'utilisation du peptide selon une des revendications 1 à 5 pour la fabrication d'une composition pharmaceutique destinée au traitement de maladies tumorales et/ou adénomateuses.

8. L'utilisation selon la revendication 6 ou 7 en présence d'un cancer des testicules, de la vessie, de l'estomac, du pancréas, du sein et/ou du rein.

9. L'utilisation selon la revendication 7 ou 8, **se caractérisant par** l'utilisation du peptide et d'un adjuvant.

10. L'utilisation selon la revendication 7 ou 8, **se caractérisant par** l'utilisation du peptide lié à une cellule présentatrice d'antigène.

11. L'utilisation du peptide selon une des revendications 1 à 5 pour la fabrication d'un anticorps.

12. Une composition pharmaceutique comprenant ce peptide selon une des revendications 1 à 5.

13. Une molécule d'acide nucléique, codante pour le peptide selon une des revendications 1 à 5.

14. Un vecteur comprenant la molécule d'acide nucléique selon la revendication 13.

15. Une cellule qui, grâce à la molécule d'acide nucléique selon la revendication 13 ou grâce au vecteur selon la revendication 14, a été génétiquement modifiée de telle manière qu'elle produit un peptide selon une des revendications 1 à 5.


### SEQUENZPROTOKOLL

<110> Immatics Biotechnologies GmbH
<120> An MHC-Moleküle bindende Tumor-assoziierte Peptide
<130> FB15156/B
<160> 81
<170> PatentIn version 3.1
<210> 1
   <211> 9
   <212> PRT
   <213> Homo sapiens
<400> 1
   Tyr Val Asp Pro Val Ile Thr Ser Ile
   1               5
<210> 2
   <211> 9
   <212> PRT
   <213> Homo sapiens
<400> 2
   Ser Val Ala Ser Thr Ile Thr Gly Val
   1               5
<210> 3
   <211> 9
   <212> PRT
   <213> Homo sapiens
<400> 3
   Ala Leu Leu Asn Ile Lys Val Lys Leu
   1 5
<210> 4
   <211> 9
   <212> PRT
   <213> Homo sapiens
<400> 4
   Ala Leu Phe Asp Gly Asp Pro His Leu
   1 5
<210> 5
   <211> 9
   <212> PRT
   <213> Homo sapiens
<400> 5
   Arg Leu Leu Asp Tyr Val Val Asn Ile
   1 5
<210> 6
   <211> 9
   <212> PRT
   <213> Homo sapiens
<400> 6
   Ala Leu Ala Asn Gly Ile Glu Glu Val
   1 5
<210> 7
   <211> 9
   <212> PRT
   <213> Homo sapiens
<400> 7
   Gln Leu Ile Asp Lys Val Trp Gln Leu
   1 5
<210> 8
   <211> 9
   <212> PRT
   <213> Homo sapiens
<400> 8
   Ala Leu Ser Asp Leu Glu Ile Thr Leu
   1 5
<210> 9
   <211> 9
   <212> PRT
   <213> Homo sapiens
<400> 9
   Ile Leu Asp Thr Gly Thr Ile Gln Leu
   1 5
<210> 10
   <211> 10
   <212> PRT
   <213> Homo sapiens
<400> 10
   Ser Leu Leu Gly Gly Asp Val Val Ser Val
   1 5 10
<210> 11
   <211> 9
   <212> PRT
   <213> Homo sapiens
<400> 11
   Phe Leu Asp Gly Asn Glu Leu Thr Leu
   1 5
<210> 12
   <211> 9
   <212> PRT
   <213> Homo sapiens
<400> 12 <210> 13
   <211> 9
   <212> PRT
   <213> Homo sapiens
<400> 13 <210> 14
   <211> 9
   <212> PRT
   <213> Homo sapiens
<400> 14
<210> 15
   <211> 9
   <212> PRT
   <213> Homo sapiens
<400> 15
   Phe Leu Leu Asp Lys Lys Ile Gly Val
   1 5
<210> 16
   <211> 9
   <212> PRT
   <213> Homo sapiens
<400> 16
   Phe Leu Asp Gly Asn Glu Met Thr Leu
   1 5
<210> 17
   <211> 9
   <212> PRT
   <213> Homo sapiens
<400> 17
   Ala Ile Val Asp Lys Val Pro Ser Val
   1 5
<210> 18
   <211> 10
   <212> PRT
   <213> Homo sapiens
<400> 18
   Asp Val Ala Ser Val Ile Val Thr Lys Leu
   1 5 10
<210> 19
   <211> 8
   <212> PRT
   <213> Homo sapiens
<400> 19
   Leu Ala Ser Val Ser Thr Val Leu
   1 5
<210> 20
   <211> 9
   <212> PRT
   <213> Homo sapiens
<400> 20
   Val Met Ala Pro Arg Thr Leu Val Leu
   1 5
<210> 21
   <211> 9
   <212> PRT
   <213> Homo sapiens
<400> 21
   Leu Leu Phe Asp Arg Pro Met His Val
   1 5
<210> 22
   <211> 10
   <212> PRT
   <213> Homo sapiens
<400> 22
   Met Thr Ser Ala Leu Pro Ile Ile Gln Lys
   1 5 10
<210> 23
   <211> 10
   <212> PRT
   <213> Homo sapiens
<400> 23
   Met Ala Gly Asp Ile Tyr Ser Val Phe Arg
   1 5 10
<210> 24
   <211> 10
   <212> PRT
   <213> Homo sapiens
<400> 24
   Glu Thr Ile Pro Leu Thr Ala Glu Lys Leu
   1 5 10
<210> 25
   <211> 10
   <212> PRT
   <213> Homo sapiens
<400> 25
   Asp Val Met Val Gly Pro Phe Lys Leu Arg
   1 5 10
<210> 26
   <211> 9
   <212> PRT
   <213> Homo sapiens
<400> 26
   Thr Ile Ile Asp Ile Leu Thr Lys Arg
   1 5
<210> 27
   <211> 9
   <212> PRT
   <213> Homo sapiens
<400> 27
   Thr Ile Val Asn Ile Leu Thr Asn Arg
   1               5
<210> 28
   <211> 9
   <212> PRT
   <213> Homo sapiens
<400> 28
   Thr Ile Ile Asp Ile Ile Thr His Arg
   1               5
<210> 29
   <211> 10
   <212> PRT
   <213> Homo sapiens
<400> 29
   Ser Ile Phe Asp Gly Arg Val Val Ala Lys
   1               5                   10
<210> 30
   <211> 9
   <212> PRT
   <213> Homo sapiens
<400> 30
   Ser Thr Ile Glu Tyr Val Ile Gln Arg
   1               5
<210> 31
   <211> 10
   <212> PRT
   <213> Homo sapiens
<400> 31
   Glu Leu Ile Lys Pro Pro Thr Ile Leu Arg
   1               5                   10
<210> 32
   <211> 11
   <212> PRT
   <213> Homo sapiens
<400> 32
   Glu Ile Ala Met Ala Thr Val Thr Ala Leu Arg
   1 5 10
<210> 33
   <211> 9
   <212> PRT
   <213> Homo sapiens
<400> 33
   Glu Thr Ile Gly Glu Ile Leu Lys Lys
   1 5
<210> 34
   <211> 9
   <212> PRT
   <213> Homo sapiens
<400> 34
   Ser Leu Ala Asp Ile Met Ala Lys Arg
   1 5
<210> 35
   <211> 9
   <212> PRT
   <213> Homo sapiens
<400> 35
   Glu Glu Ile Ala Phe Leu Lys Lys Leu
   1 5
<210> 36
   <211> 9
   <212> PRT
   <213> Homo sapiens
<400> 36
   Asp Glu Ala Ala Phe Leu Glu Arg Leu
   1 5
<210> 37
   <211> 8
   <212> PRT
   <213> Homo sapiens
<400> 37
   Asp Glu Met Lys Val Leu Val Leu
   1 5
<210> 38
   <211> 8
   <212> PRT
   <213> Homo sapiens
<400> 38
   Asp Glu Val Lys Phe Leu Thr Val
   1 5
<210> 39
   <211> 9
   <212> PRT
   <213> Homo sapiens
<400> 39
   Asn Glu Asn Ser Leu Phe Lys Ser Leu
   1 5
<210> 40
   <211> 8
   <212> PRT
   <213> Homo sapiens
<400> 40
   Asp Glu Phe Lys Val Val Val Val
   1 5
<210> 41
   <211> 9
   <212> PRT
   <213> Homo sapiens
<400> 41
   Glu Glu Val Lys Leu Ile Lys Lys Met
   1 5
<210> 42
   <211> 9
   <212> PRT
   <213> Homo sapiens
<400> 42
   Asp Glu Val Lys Leu Pro Ala Lys Leu
   1 5
<210> 43
   <211> 9
   <212> PRT
   <213> Homo sapiens
<400> 43
   Thr Glu Arg Glu Leu Lys Val Ala Tyr
   1 5
<210> 44
   <211> 10
   <212> PRT
   <213> Homo sapiens
<400> 44
   Asn Glu Phe Ser Leu Lys Gly Val Asp Phe
   1 5 10
<210> 45
   <211> 9
   <212> PRT
   <213> Homo sapiens
<400> 45
   Asn Glu Gln Asp Leu Gly Ile Gln Tyr
   1 5
<210> 46
   <211> 8
   <212> PRT
   <213> Homo sapiens
<400> 46
   Glu Glu Arg Ile Val Glu Leu Phe
   1 5
<210> 47
   <211> 10
   <212> PRT
   <213> Homo sapiens
<400> 47
   Glu Glu Ile Arg Glu Ala Phe Arg Val Phe
   1 5 10
<210> 48
   <211> 9
   <212> PRT
   <213> Homo sapiens
<400> 48
   Asp Glu Tyr Ile Tyr Arg His Phe Phe
   1 5
<210> 49
   <211> 9
   <212> PRT
   <213> Homo sapiens
<400> 49
   Asp Glu Leu Glu Leu His Gln Arg Phe
   1 5
<210> 50
   <211> 9
   <212> PRT
   <213> Homo sapiens
<400> 50
   Ser Glu Val Lys Phe Thr Val Thr Phe
   1 5
<210> 51
   <211> 8
   <212> PRT
   <213> Homo sapiens
<400> 51
   Ile Glu Thr Ile Ile Asn Thr Phe
   1               5
<210> 52
   <211> 9
   <212> PRT
   <213> Homo sapiens
<400> 52
   Lys Glu Asn Pro Leu Gln Phe Lys Phe
   1               5
<210> 53
   <211> 8
   <212> PRT
   <213> Homo sapiens
<400> 53
   Asp Glu Val Arg Thr Leu Thr Tyr
   1               5
<210> 54
   <211> 9
   <212> PRT
   <213> Homo sapiens
<400> 54
   Gly Glu Ala Val Val Asn Arg Val Phe
   1               5
<210> 55
   <211> 10
   <212> PRT
   <213> Homo sapiens
<400> 55
   Glu Glu Val Leu Ile Pro Asp Gln Lys Tyr
   1               5                   10
<210> 56
   <211> 9
   <212> PRT
   <213> Homo sapiens
<400> 56
   Asp Glu Gly Arg Leu Val Leu Glu Phe
   1 5
<210> 57
   <211> 8
   <212> PRT
   <213> Homo sapiens
<400> 57
   Asp Glu Val Glu Leu Ile His Phe
   1 5
<210> 58
   <211> 9
   <212> PRT
   <213> Homo sapiens
<400> 58
   Val Glu Val Leu Leu Asn Tyr Ala Tyr
   1 5
<210> 59
   <211> 9
   <212> PRT
   <213> Homo sapiens
<400> 59
   Thr Glu Asn Asp Ile Arg Val Met Phe
   1 5
<210> 60
   <211> 8
   <212> PRT
   <213> Homo sapiens
<400> 60
   Leu Glu Gly Leu Thr Val Val Tyr
   1 5
<210> 61
   <211> 8
   <212> PRT
   <213> Homo sapiens
<400> 61
   Asn Glu Leu Pro Thr Val Ala Phe
   1 5
<210> 62
   <211> 9
   <212> PRT
   <213> Homo sapiens
<400> 62
   Glu Glu Phe Gly Gln Ala Phe Ser Phe
   1 5
<210> 63
   <211> 8
   <212> PRT
   <213> Homo sapiens
<400> 63
   Val Glu Ala Ile Phe Ser Lys Tyr
   1 5
<210> 64
   <211> 9
   <212> PRT
   <213> Homo sapiens
<400> 64
   Asp Glu Arg Thr Phe His Ile Phe Tyr
   1 5
<210> 65
   <211> 9
   <212> PRT
   <213> Homo sapiens
<400> 65
   Thr Glu Lys Val Leu Ala Ala Val Tyr
   1 5
<210> 66
   <211> 9
   <212> PRT
   <213> Homo sapiens
<400> 66
   Val Glu Ser Pro Leu Ser Val Ser Phe
   1 5
<210> 67
   <211> 10
   <212> PRT
   <213> Homo sapiens
<400> 67
   Ser Glu Ala Gly Ser His Thr Leu Gln Trp
   1 5 10
<210> 68
   <211> 8
   <212> PRT
   <213> Homo sapiens
<400> 68
   Asp Glu Gly Lys Val Ile Arg Phe
   1 5
<210> 69
   <211> 9
   <212> PRT
   <213> Homo sapiens
<400> 69
   Ala Leu Ala Ala Val Val Thr Glu Val
   1 5
<210> 70
   <211> 9
   <212> PRT
   <213> Homo sapiens
<400> 70
   Thr Leu Ile Glu Asp Ile Leu Gly Val
   1 5
<210> 71
   <211> 9
   <212> PRT
   <213> Homo sapiens
<400> 71
   Ala Leu Phe Gly Ala Leu Phe Leu Ala
   1 5
<210> 72
   <211> 9
   <212> PRT
   <213> Homo sapiens
<400> 72
   Val Leu Ala Thr Leu Val Leu Leu Leu
   1 5
<210> 73
   <211> 9
   <212> PRT
   <213> Homo sapiens
<400> 73
   Thr Leu Asp Asp Leu Ile Ala Ala Val
   1 5
<210> 74
   <211> 9
   <212> PRT
   <213> Homo sapiens
<400> 74
   Tyr Leu Asp Asn Gly Val Val Phe Val
   1 5
<210> 75
   <211> 9
   <212> PRT
   <213> Homo sapiens
<400> 75
   Ser Val Phe Ala Gly Val Val Gly Val
   1 5
<210> 76
   <211> 10
   <212> PRT
   <213> Homo sapiens
<400> 76
   Ser Leu Ile Asn Val Gly Leu Ile Ser Val
   1 5 10
<210> 77
   <211> 9
   <212> PRT
   <213> Homo sapiens
<400> 77
   Ala Leu Ala Asp Gly Val Gln Lys Val
   1 5
<210> 78
   <211> 9
   <212> PRT
   <213> Homo sapiens
<400> 78
   Thr Tyr Gly Glu Ile Phe Glu Lys Phe
   1 5
<210> 79
   <211> 9
   <212> PRT
   <213> Homo sapiens
<400> 79
   Tyr Tyr Met Ile Gly Glu Gln Lys Phe
   1 5
<210> 80
   <211> 10
   <212> PRT
   <213> Homo sapiens
<400> 80
   Glu Leu Thr Leu Gly Glu Phe Leu Lys Leu
   1 5
<210> 81
   <211> 9
   <212> PRT
   <213> Human Immunodeficiency Virus
<400> 81
   Ile Leu Lys Glu Pro Val His Gly Val
   1 5